# EUROPEAN PATENT APPLICATION

(11) **EP 4 497 383 A1**
(43) Date of publication of application: **29.01.2025**
(21) Application number: 24187343.9
(22) Date of filing: 09.07.2024
(51) Int. Cl.: A61B 5/16, G16H 20/70, G16H 50/20

(54) **PROVIDING TRAINING THROUGH PRESENTATION OF VISUAL ELEMENTS TO ADDRESS MALADAPTIVE BEHAVIOR OF USERS WITH SKIN PATHOLOGIES**

(30) Priority: 03.11.2023 US 202363547286 P; 28.07.2023 US 202363529503 P; 19.06.2024 US 202418747999
(71) Applicant: Click Therapeutics, Inc., New York, NY 10013 (US)
(72) Inventor: ADLER, Samantha, New York 10013 (US); SNIPES, Cassandra, New York 10013 (US)
(74) Representative: Marks & Clerk LLP

(57) **Abstract**

Provided herein are systems and methods for providing training via visual elements for presentation to address maladaptive behavior in users. A server may identify a session defining a plurality of tasks for a user to be performed using at least one hand of the user holding a display to address a propensity for behavior associated with a condition of the user. The server may provide a first indication to perform a first task using the at least one hand. The server may provide, subsequent to the first task, a second indication to perform a second task using the at least one hand. The server may provide, subsequent to the second task, a third indication to perform a third task using the at least one hand. The user may be administered with an effective amount of a medication at least partially concurrently with the performance of the tasks.

## Description

### CROSS REFERENCES TO RELATED APPLICATIONS

The present application claims priority to U.S. Provisional Patent Application No. 63/529,503, titled "Providing Training Through Presentation of Visual Elements to Address Maladaptive Behavior of Users with Skin Pathologies," filed July 28, 2023 and U.S. Provisional Patent Application No. 63/547,286, titled "Providing Training Through Presentation of Visual Elements to Address Maladaptive Behavior of Users with Skin Pathologies," filed November 3, 2023, each of which is incorporated herein by reference in their entireties.

### BACKGROUND

Skin pathologies may lead to pruritus in an affected region of the skin, causing a subject to reflexively scratch the affected region. The urge to scratch at skin made itchy by a skin pathology may become too difficult for individuals to resist. Scratching at these ailments with the intent to calm the symptoms, while possibly momentarily relieving, can cause further skin problems and further exacerbating the skin pathology. This maladaptive behavior (e.g., scratching) can become a nuisance for the individual experiencing the symptoms by interrupting the individual's ordinary activities. Scratching may also harm the skin. For example, scratching at psoriatic lesions may cause the psoriasis to worsen, or may break the skin barrier and draw blood. Further complications from scratching can include skin infections, flaking of skin, or discoloration of skin.

Under pharmaceutical approaches, medications may be taken by an individual to lessen the itch severity from the skin pathology. Preventative medications also exist that help minimize the likelihood that an individual will experience itchiness. These medications may require prescriptions and may be oral or topical. Topical and oral medications may be inconvenient or embarrassing for an individual to continuously administer, especially if the itchiness occurs while the individual is in public or on an immodest body part of the individual. Sometimes, the individual may have to wait to administer a medication due to the individual's location or the location of the skin pathology. Furthermore, sometimes the individual may not have a prescription for the skin pathology or may currently be without medication. Experiencing itchiness for any period of time may cause extreme discomfort to the individual, and the propensity of the individual to maladaptively scratch may become too much to bear.

In addition, therapeutic exercises to mitigate the effects of itching may lessen the severity of the maladaptive scratching. Certain behavioral therapies may aid an individual in reducing or removing the urge to scratch. Access to these behavioral therapies can be difficult to obtain, especially in the moment when an individual feels the urge to scratch. Furthermore, the therapies may not prove useful for the individual without consistent adherence. Lastly, it can be difficult to ascertain which therapies would be the most beneficial to an individual or if a combination of therapies would be the most beneficial.

### SUMMARY

To resolve these and other technical challenges, a service may provide a series of interventions for digital therapeutics aimed at reducing a user's frequency, intensity, or propensity towards a maladaptive behavior associated with a skin pathology. The series of tasks are a combination of inhibitory control training, habit reversal therapy (HRT), and urge surfing. By using the combination of these three tasks through a computing device (e.g., a user's smart phone), the user's ability to control the maladaptive behavior or urge may be increased in a synergistic manner. The tasks may contain different actions to be performed by the user, such as via an end user device associated with the user. The objective of each different task and its corresponding actions can be to resolve or reduce a subject's frequency, intensity, or propensity for a maladaptive behavior (e.g., to react to an itch on the skin). The service can identify and select a task based on a sequential ordering of tasks, or based on a request initiated by the user via a device of the user.

By using a combination of go/no-go training, habit reversal therapy (HRT), and urge surfing through a computing device (e.g., a smartphone), the user's ability to control the maladaptive behavior or urge may be increased in a synergistic manner. Controlling the maladaptive urge can be a facet of remediating or resolving symptoms of a skin pathology such as atopic dermatitis or psoriasis. Under the go/no-go-training, the user can be directed to perform an action and then subsequently refrain from performing an action, thereby priming the user for neuroplasticity.

The combination of these interventions can lead to synergy by unexpectedly combining two opposite approaches: a top-down approach using habit reversal therapy and urge surfing and a bottom-up approach using inhibitory control. The top-down approach may refer to intentional, observable training, by giving users a set of conscious tools to decide how to engage in a response rather than succumbing to the normal automatic response to give in to an urge. The bottom-up approach may refer to subconscious training to train the user to subconsciously improve the user's ability to control response to maladaptive behaviors. In addition, by having the user exposed to the combination of these interventions, the efficacy of the medication that the user is taking for the skin pathology may be improved or enhanced as the user becomes less likely to partake in the maladaptive behavior (e.g., scratching).

The user may adapt to pay less attention to the maladaptive urge or to resist the urge. Habit reversal therapy centers around directing the user to realize that resisting an urge may lead to the urge dissipating and then replacing the maladaptive behavior with observable adaptive behavior. HRT can help to reduce unwanted repetitive behaviors by enabling the user to become aware of cues of the onset of the maladaptive behavior and then replacing this maladaptive behavior with another physically incompatible behavior. For example, instead of scratching at the skin pathology, an HRT task or action may prompt the user to pinch her fingers together. This replacement of maladaptive behavior for other preferred behaviors can lead to anxiety or unease for the user. In this case, the addition of urge surfing helps to alleviate negative feelings resulting from abstaining from the maladaptive behavior. Urge surfing can motivate the user to persist through the urge to perform the maladaptive behavior, such as by playing a game, visualizing a calming scenario, or through biofeedback such as pupil dilation or heart rate control.

A combination of these therapies can greatly reduce the propensity to perform the maladaptive behavior. As an illustrative example, the combination can include the service providing the user a game through her mobile device. The user may place her thumbs on the mobile device to control the game and to tilt the mobile device in the correct direction based on cues from the game. This illustrative example shows an alternative behavior to prevent the user from scratching, as holding the phone is an incompatible behavior, thereby incorporating HRT. To continue with the illustrative example, cues could arise during the game that prompt the user to stop the main task, such as phone tilting, and instead rest for a period of time with her hands on the mobile device. This action would incorporate a go/no-go (GNG) therapy. Furthermore, real-time biofeedback may control the game and its cues, thereby helping the user to practice mindfulness in the form of urge surfing. This combination of therapies provided by the service can lead to a reduction in performance of the maladaptive behavior.

To provide these therapies, the service may select a task and associated actions for the user to transmit to the end user device. The service may also time the delivery or presentation of the action to the user to avoid the user from having to actively access the digital therapeutics application on her device while suffering from itching. The service may time the delivery or presentation of the action to the user subsequent to the completion of a prior task. Furthermore, as the service acquires additional data about the user, the service may be able to select tasks and actions more targeted toward the specific user and her condition, and may store this data in a profile of the user. The service may select a subsequent task based on at least the prior task, the completion of the prior task, the profile of the user, or an evaluation of the user's performance on prior tasks, among others.

Since the application operates on the subject's mobile device, or at least a mobile device that she can access easily and reliably, e.g., according to the predetermined frequency (e.g., once per day), the application provides real-time support to the subject. For example, upon receiving a request from the user to initiate a session, the application initiates a session in real-time, i.e., within at least a few milliseconds from receiving the request. Such prompt guidance cannot be achieved via in-person visits, phone calls, video conferences or even text messages between the user and health care providers, such as dermatologists. In this manner, the application is able to provide and customize tasks for the user based on the performance of the user. This can create an iteratively improving service for the user wherein overall bandwidth and data communications are minimized due to the increasing usefulness of each task.

Furthermore, the user can receive tasks relating to the condition with ease to help retrain maladaptive scratching for those with skin pathologies. This may reduce or eliminate barriers to the user from physically accessing their device while combating maladaptive scratching. By selecting the tasks sent to the user to address the subject's propensity to scratch, the quality of a human computer interactions (HCI) between the user and the device may be improved. In addition, since the messages are more related to the user's condition, unnecessary consumption of computational resources (e.g., processing and memory) of the service and the user device and the network bandwidth may be reduced, relative to sending ineffective messages. Furthermore, in the context of a digital therapeutics application, the individualized selection of such tasks may result in the delivery of user-specific interventions to improve subject's adherence to the treatment. This may result not only in higher adherence to the therapeutic interventions but also potential improvements to subject's maladaptive behavior.

Aspects of the present disclosure relate to systems and methods for providing training via visual elements to address maladaptive behavior. The system may include a computing system having one or more processors coupled with memory. The computing system may identify a session defining a set of tasks for a user to be performed using at least one hand of the user holding a display to address a propensity for behavior associated with a condition of the user. The computing system may provide a first indication to perform a first task of the set of tasks using at least one hand in a first portion of the session. The computing system may provide, subsequent to the first task, a second indication to perform a second task of the set of tasks using at least one hand in a second portion of the session. The computing system may provide, subsequent to the second task, a third indication to perform a third task of the set of tasks using at least one hand in a third portion of the session.

In some embodiments, the set of tasks may include one or more of (i) a go/no-go task to prompt the user to perform or withhold an action, (ii) a habit reversal therapy task to prompt the user to perform a second action instead of performing a third action associated with the maladaptive behavior, and (iii) an urge surfing task to prompt the user to perform a fourth task for the duration of the propensity for the maladaptive behavior. In some embodiments, the computing system may provide the second indication responsive to completion of the first task, and wherein providing the third indication further comprises providing the third indication responsive to completion of the second task. In some embodiments, at least one of the tasks may be to perform at least one of an interaction or non-interaction with a stimulus associated with the condition, the stimulus comprising at least one of text, an image, or audio.

In some embodiments, the computing system may receive a request from the user indicating an onset of the behavior associated with the condition. The computing system may initiate, responsive to receiving the request, the session to address the propensity for the behavior associated with the condition. In some embodiments, the computing system may receive sensory data identifying one or more physiological measurements of the user while performing the first task. The computing system may modify the second task based on the one or more physiological measurements of the user identified in the sensory data. In some embodiments, the one or more physiological measurements may further include at least one of a breathing pattern, a pupil dilation, a heart rate, skin conductance, or oxygen saturation.

In some embodiments, the computing system may determine a score based on performance of at least one of the tasks. The score may identify at least one of (i) a degree of compliance of the user with at least one task or (ii) a likelihood of overcoming the propensity for the maladaptive behavior. In some embodiments, the computing system may generate feedback to provide to the user based on performance of at least one of the tasks. In some embodiments, the condition includes a skin condition corresponding to at least one of atopic dermatitis or psoriasis and the user is taking a medication to address the skin condition, at least in partial concurrence with the session to address the propensity of maladaptive behavior of scratching.

Aspects of the present disclosure relate to systems and methods for reducing the frequency or intensity for a maladaptive behavior related to a skin pathology in a user in need thereof. A computing system may obtain a first metric associated with the user prior to a plurality of time instances. The computing system may repeat, over each of the plurality of time instances, a presentation of a respective indication to perform a task of the plurality of tasks using the at least one hand to hold the display. The computing system may obtain a second metric associated with the user after at least one of the plurality of time instances. A reduction in the frequency or intensity for the maladaptive behavior related to the skin pathology may occur in the user when the second metric is (i) increased compared to the first metric by at least a first predetermined margin, or (ii) decreased compared to the first metric by at least a second predetermined margin.

In some embodiments, the maladaptive behavior may include at least one of (i) continuous scratching of an affected epidermal tissue of the user, (ii) an intermittent scratching of the affected epidermal tissue, or (iii) a reflexive reaction to a pruritis on the affected epidermal tissue. In some embodiments, the skin pathology may include at least one of (i) atopic dermatitis, (ii) contact dermatitis, (iii) psoriasis, (iv) urticaria, (v) prurigo nodularis, (vi) lichen planus, (vii) acne vulgaris, (viii) a drug-induced rash, (ix) xerosis, or (x) uremia. In some embodiments, the skin pathology may be caused by at least one of an infection, an allergy, or a genetic mutation.

In some embodiments, the reduction may occur when the second metric is increased compared to the first metric by the first predetermined threshold margin, and the first metric and the second metric may be dermatology life quality index (DLQI) values. In some embodiments, the reduction may occur when the second metric decreased compared to the first metric by the second predetermined threshold margin, and the first metric and the second metric may be peak pruritus numerical rating scale (PP-NRS) values. In some embodiments, the reduction may occur when the second metric increased compared to the first metric by the first predetermined threshold margin, and the first metric and the second metric may be patient-reported outcomes measurements information systems (PROMIS) values.

In some embodiments, the reduction may occur when the second metric decreased compared to the first metric by the second predetermined threshold margin, and wherein the first metric and the second metric are patient-oriented eczema measure (POEM) values. In some embodiments, the reduction may occur when the second metric decreased compared to the first metric by the second predetermined threshold margin, and wherein the first metric and the second metric may be a psoriasis symptom inventory (PSI) values. In some embodiments, the reduction may occur when the second metric decreased compared to the first metric by the second predetermined threshold margin, and wherein the first metric and the second metric may be Sleep-Related Itch and Scratch Scale (SRISS) values. In some embodiments, the reduction may occur when the second metric decreased compared to the first metric by the second predetermined threshold margin, and wherein the first metric and the second metric may be Scratch Intensity and Impact Scale (SIIS) values. In some embodiments, the first metric and the second metric may be computerized cognitive assessment values.

In some embodiments, the user may be an adult diagnosed with the skin pathology and is associated with the first metric satisfying a baseline threshold. In some embodiments, the first metric may be an itch numerical ration scale (NRS) less than or equal to the baseline threshold of 4. In some embodiments, the user may be taking a medication to address the skin pathology, at least in partial concurrence with the first time instance or second plurality of time instance. In some embodiments, the medication may include at least one of capsaicin, diflorasone, diphenhydramine, doxepin, hydrocortisone, prednisone, prednicarbate, pimecrolimus, tetracaine, tacrolimus, terbinafine, or dexamethasone (zema lotion), among others.

In some embodiments, the plurality of tasks may include (i) a go/no-go task to prompt the user to perform or withhold a first action, (ii) a habit reversal therapy task to prompt the user to perform a second action instead of performing a third action associated with the maladaptive behavior, and (iii) an urge surfing task to perform a third action for the duration of the maladaptive behavior. In some embodiments, the task for a time instance of the plurality of time instances may be modified based on response data from the user while performing another task in a prior time instance before the time instance.

In some embodiments, the task for a time instance of the plurality of time instances may be modified based on a score determined from performing another task of the plurality of tasks in a prior time instance before first time instance. In some embodiments, the task for the time instance may be modified to change an order of performance, a time length to complete the task, or a difficulty of performing the task. In some embodiments, the task for a time instance of the plurality of time instances may be independent of another task in a prior time instance before the first time instance. In some embodiments, the plurality of time instances ranges between 2 weeks to 6 months. In some embodiments, at least one of the first predetermined margin or a second predetermined margin may be based on metrics associated with a second user provided with a control therapy. In some embodiments, the user may belong to a treatment group and the second user may belong to a control group.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system for providing training via visual elements for presentation to address maladaptive behavior in users in accordance with an illustrative embodiment;
FIG. 2 depicts a block diagram for a process to identify a session, receive a request indicating the onset of the behavior and to provide an indication to perform a task in the system for providing training in accordance with an illustrative embodiment;
FIG. 3 depicts a block diagram for a process to assess user performance based on response data in the system for providing training in accordance with an illustrative embodiment;
FIG. 4 depicts a block diagram for a process to provide feedback to the user and to determine a task based on a performance of a task in the system for providing training in accordance with an illustrative embodiment;
FIGs. 5A-B depict a flow diagram of a method of providing training via visual elements for presentation to address maladaptive behavior in users in accordance with an illustrative embodiment;
FIG. 6 depicts a block diagram of an environment for presenting a task on a device in the system for providing training via visual elements for presentation to address maladaptive behavior in users in accordance with an illustrative embodiment;
FIGs. 7A-7C depict example user interfaces of various types of interventions in the system for providing training to address maladaptive behaviors in users with skin pathologies in accordance with an illustrative embodiment;
FIGs. 8A-8B depict screenshots of example user interfaces of urge surfing training for a system for providing training to address maladaptive behavior in users in accordance with an illustrative embodiment;
FIGs. 9A-9C depict screenshots of example user interfaces of urge surfing training for a system for providing training via visual elements for presentation to address maladaptive behavior in users in accordance with an illustrative embodiment;
FIGs. 10A-10B depict screenshots of example user interfaces of go/no-go training for a system for providing training via visual elements for presentation to address maladaptive behavior in users in accordance with an illustrative embodiment;
FIG. 11 depicts a screenshot of an example user interface of go/no-go training in the system for providing training via visual elements for presentation to address maladaptive behavior in users in accordance with an illustrative embodiment;
FIG. 12 depicts a screenshot of an example user interface of go/no-go training in the system for providing training to address maladaptive behaviors in users with skin pathologies in accordance with an illustrative embodiment;
FIG. 13 depicts a screenshot of an example user interface in the system for providing training to address maladaptive behaviors in users with skin pathologies in accordance with an illustrative embodiment;
FIGs. 14A-D depict screenshots of example user interfaces of go/no-go training in the system for providing training via visual elements for presentation to address maladaptive behavior in users in accordance with an illustrative embodiment
FIGs. 15A-D depict screenshots of example user interfaces of go/no-go training in the system for providing training via visual elements for presentation to address maladaptive behavior in users in accordance with an illustrative embodiment
FIG. 16 depicts a screenshot of an example user interface with options for various interventions in the system for providing training via visual elements for presentation to address maladaptive behavior in users in accordance with an illustrative embodiment;
FIGs. 17A-M depict screenshots of example user interfaces for urge surfing training in the system for providing training via visual elements for presentation to address maladaptive behavior in users in accordance with an illustrative embodiment;
FIG. 18 depicts a flow diagram of a method of reducing a frequency or intensity for a maladaptive behavior related to a skin pathology in a user in need thereof in accordance with an illustrative embodiment;
FIG. 19 depicts a timeline of the study design for evaluating inhibitory control, habit reversal therapy and urge surfing to disrupt scratching for subjects with skin pathologies; and
FIG. 20 is a block diagram of a server system and a client computer system in accordance with an illustrative embodiment.

### DETAILED DESCRIPTION

For purposes of reading the description of the various embodiments below, the following enumeration of the sections of the specification and their respective contents may be helpful:
Section A describes systems and methods for providing training via visual elements for presentation to address maladaptive behavior in users;
Section B describes systems and methods for reducing frequencies or intensities for maladaptive behaviors related to skin pathologies in users;
Section C describes a network and computing environment which may be useful for practicing embodiments described herein.

### A. Systems and Methods for Providing training via visual elements for Presentation to address maladaptive behavior in users

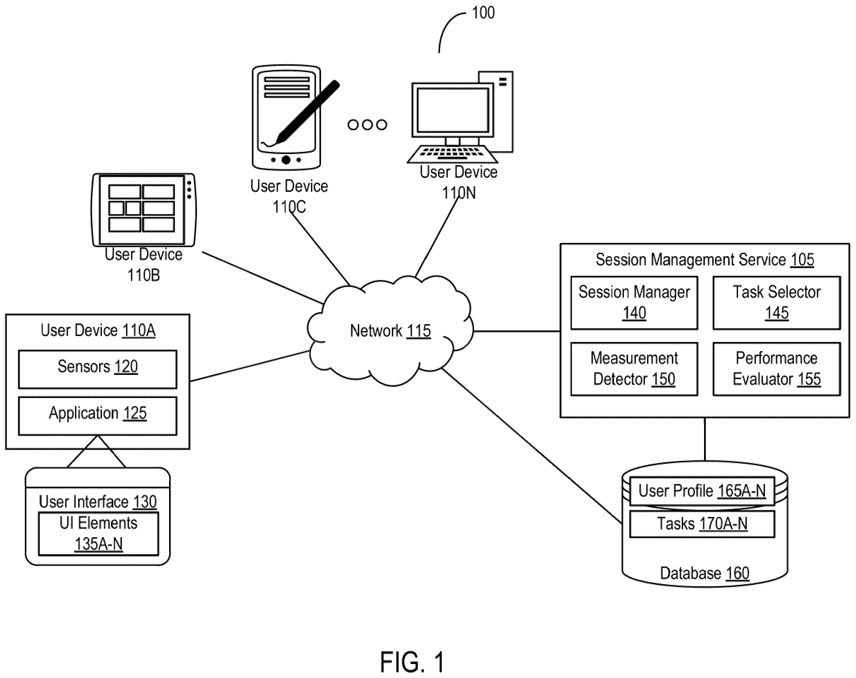

Referring now to FIG. 1, depicted is a block diagram of a system 100 for providing training via visual elements for presentation to address maladaptive behavior in users. In an overview, the system 100 may include at least one session management service 105 and a set of user devices 110A-N (hereinafter generally referred to as user devices 110 or client devices 110), communicatively coupled with one another via at least one network 115. At least one user device 110 (e.g., the first user device 110A as depicted) may include at least one sensor 120 and at least one application 125. The application 125 may include or provide at least one user interface 130 with one or more user interface (UI) elements 135A-N (hereinafter generally referred to as UI elements 135). The session management service 105 may include at least one session manager 140, one task selector 145, one measurement detector 150, or at least one performance evaluator 155, among others. The session management service 105 may include or have access to at least one database 160. The database 160 may store, maintain, or otherwise include one or more user profiles 165A-N (hereinafter generally referred to as user profiles 165) and one or more tasks 170A-N among others. The functionality of the application 125 may be performed in part on the session management service 105.

In further detail, the session management service 105 may (sometimes herein generally referred to as a computing system or a service) be any computing device comprising one or more processors coupled with memory and software and capable of performing the various processes and tasks described herein. The session management service 105 may be in communication with the one or more user devices 110 and the database 160 via the network 115. The session management service 105 may be situated, located, or otherwise associated with at least one server group. The server group may correspond to a data center, a branch office, or a site at which one or more servers corresponding to the session management service 105 is situated. The session management service 105 may be situated, located, or otherwise associated with one or more of the client devices 110. Some components of the session management service 105 may be located within the server group and some may be located within the client device. For example, the session manager 140 may operate or be situated on the user device 110A, and the task selector 145 may be operate or be situated on the server group.

Within the session management service 105, the session manager 140 may identify a session defining a set of tasks 170 for a user to perform by the application 125 on respective user devices 110. The task selector 145 may select at least one task 170 to provide via the session manager 140 to the user device 110. The task selector 145 may select the task 170 based on a performance of a prior selected task. The measurement detector 150 may receive sensory data from the user via the user device 110 and may modify a task 170 or a selection of the task 170 based on measurements included in the sensory data. The performance evaluator 155 may determine a score based on a performance of one or more tasks 170 and may provide feedback to the user via the application 125 presenting on the user device 110.

The user device 110 (sometimes herein referred to as an end user computing device or client device) may be any computing device comprising one or more processors coupled with memory and software and capable of performing the various processes and tasks described herein. The user device 110 may be in communication with the session management service 105 and the database 160 via the network 115. The user device 110 may be a smartphone, other mobile phone, tablet computer, wearable computing device (e.g., smart watch, eyeglasses), or laptop computer. The user device 110 may be used to access the application 125. In some embodiments, the application 125 may be downloaded and installed on the user device 110 (e.g., via a digital distribution platform). In some embodiments, the application 125 may be a web application with resources accessible via the network 115.

In some embodiments, the user device 110 may be coupled with at least one sensor 120. The sensors 120 may provide sensory data to the user device 110 or the session management service 105 regarding the user or the user's performance. The data may include biofeedback. Biofeedback can include various physical attributes of the user, such as the user's voice, heart rate, pupil dilation, breathing pattern, blood oxygen saturation, or response time, among others. The sensors 120 may sense, detect, or identify data related to the user or her performance. In some embodiments, the sensors 120 may include audio sensors, such as a microphone (e.g., piezoelectric microphone, ribbon microphone, among others) or decibel meter. The audio sensors 120 may detect a voice, heartbeat, or other audio indication from the user. In some embodiments, the sensors 120 may include imaging sensors such as cameras or video cameras. The imaging sensors 120 may include photodiodes, active-pixel sensors, charge-coupled devices, or quanta image sensors, among others. In some embodiments, the sensors 120 may include timers. In some embodiments, the sensors 120 may include light sensors, such as photodiodes, photoresistors, or phototransistors.

Continuing on, the sensors 120 may include one or more pulse oximeters to detect an oxygen saturation of the user's blood. In some embodiments, the sensors 120 can detect a response time for the user to interact with the user device 110 based on a timer, imaging sensors, or the other sensors 120. In some embodiments, biofeedback may be captured by the sensors 120. For example, an imaging sensor 120 may detect a pupil dilation of the user, or a heartrate sensor may detect a pulse frequency or a change in heartrate of the user. For example, an audio sensor may detect a breathing pattern of the user. Data accumulated from the sensors 120 may be processed by the session management service 105 or the application 125.

The application 125 executing on the user device 110 may be a digital therapeutics application and may provide a session (sometimes herein referred to as a therapy session) to address the propensity for a maladaptive behavior associated with a condition of the user. The behavior may include maladaptive scratching prompted by a condition such as a skin pathology. A skin pathology may cause other maladaptive behaviors, such as skin picking, pinching, or rubbing. Other behaviors may cause or be related to a condition of the user. The application 125 may be used to present tasks prompting the user to perform actions to reduce the frequency, intensity, or propensity to participate in the behavior associated with the condition of the user. The tasks may be presented to the user as a result of sending a request to begin a session, detected measurements of the user received from the client device, or a scheduled time or period, among others.

The user of the application 125 may be suffering from or at risk of a skin pathology. The skin pathology may be, for example, atopic dermatitis, contact dermatitis, psoriasis, urticaria, prurigo nodularis, lichen planus, acne vulgaris, a drug-induced rash, xerosis, uremia, non-contact dermatitis (eczema), an allergic reaction (such as to a plant, medication, or other exposure), a burn, rash, scabies, hives, or insect bites, among others. The user may participate in maladaptive or other behaviors to attempt to reduce or mitigate the effects of the skin pathology. For example, the user may scratch, pick at, rub, or otherwise physically stimulate the skin affected by the pathology. The user may be at least partially concurrently taking medication to address the pathology, while being provided sessions through the application 125. The medication may be at least orally administered or topically applied. For instance, the user may be taking or applying capsaicin, diflorasone, diphenhydramine, doxepin, hydrocortisone, prednisone, prednicarbate, pimecrolimus, tetracaine, tacrolimus, terbinafine, corticosteroids, vitamin D analogues, retinoids, calcineurin inhibitors, immunosuppressants, or antihistamines, among others. The medication may be of an effective amount (e.g., dosage or frequency), which may be sufficient to effect positive outcomes or reduction in symptoms (e.g., reduction in itch or propensity to scratch). The efficacy of the medication in terms of addressing the user's maladaptive behavior may be improved or enhanced when taken, at least partially concurrently with the use of the application 125.

The skin pathologies described herein may be treated utilizing a variety of medications or treatment protocols depending upon the specific skin pathology. For example, treatment or therapeutic options may include, but are not limited to behavioral therapy, avoidance therapy, hormone replacement therapy (HRT), antibiotics, antihistamines, laser skin resurfacing, medicated creams, ointments or gels, moisturizers, oral medications, steroid pills, creams or injections, surgical procedures, skin cleansers, and lifestyle changes, among others.

Atopic dermatitis, also known as atopic eczema, refers to a non-contagious long-term type of skin inflammation. The cause of atopic dermatitis is believed to involve genetics, immune system dysfunction, and environmental exposure. Exposure to certain chemicals or irritants may exacerbate symptoms of atopic dermatitis. Various treatments for atopic dermatitis include enhancing the skin barrier with moisturizing creams, anti-inflammatory corticosteroid creams, dietary exclusion, phototherapy, calcineurin inhibitors, and antihistamines, among others.

Contact dermatitis refers to a type of non-contagious acute or chronic inflammation of the skin caused by chemical or physical agents. Symptoms of contact dermatitis include itchy or dry skin, rashes, bumps, blisters or swelling. Contact dermatitis is caused by prolonged exposure to irritants, leading to direct injury of the epidermal cells of the skin, which activates an immune response, resulting in an inflammatory cutaneous reaction.

Psoriasis refers to a skin disease that causes a rash with itchy, scaly patches, most commonly on the knees, elbows, trunk and scalp. Psoriasis is a common, chronic disease with no cure. It can be painful, interfere with sleep and make it hard to concentrate. The condition tends to go through cycles, flaring for a few weeks or months, then subsiding for a while. Common triggers in people with a genetic predisposition to psoriasis include infections, cuts or burns, and certain medications. The five main types of psoriasis are plaque, guttate, inverse, pustular, and erythrodermic. Various treatments include steroid creams, vitamin D3 cream, ultraviolet light, immunosuppressive drugs, such as methotrexate, and biologic therapies targeting specific immunologic pathways, among others.

Urticaria or hives refers to a kind of an acute form skin rash with red, raised, itchy bumps that may burn or sting. Hives frequently occur following an infection or as a result of an allergic reaction such as to medication, insect bites, or food. Other causes of hives may include psychological stress, hay fever, asthma, or cold temperature. Treatments may include avoidance of allergens, antihistamines, corticosteroids, or leukotriene inhibitors. Immunosuppressants are used in long-term, severe cases.

Prurigo nodularis refers to a chronic skin disorder characterized by itchy, nodular lesions typically appearing on the trunk, arms and legs. Prurigo nodularis is often associated with other dermatologic conditions such as atopic dermatitis. Other known causes are autoimmune diseases, thyroid disease, lymphoma, and kidney failure. Treatments include steroids, dupilumab, vitamins, cryosurgery, thalidomide and UVB light, among others.

Lichen planus refers to a chronic inflammatory and autoimmune disorder that affects the skin, nails, hair, and mucous membranes. Lichen planus is characterized by polygonal, flat-topped, violaceous papules and plaques with overlying, reticulated, fine white scale (Wickham's striae), commonly affecting dorsal hands, flexural wrists and forearms, trunk, anterior lower legs and oral mucosa, and may include popular, annular, linear, hypertrophic, atrophic, bullous, actinic, ulcerative, pigmented, follicular, or inverse lesions, among others.

Xerosis, also no as dry skin, refers to a common condition affecting skin moisture and barrier function. Causes of dry skin include abnormalities in the barrier function of the stratum corneum of the skin. These abnormalities may be caused by dry climates, excessive heat, environmental or chemical irritants, burns, autoimmune disorders, and abrasions, among others.

Acne vulgaris refers to a chronic skin condition caused by the clogging of hair follicles along the surface of the skin. Acne can be classified as mild, moderate, or severe to determine an appropriate treatment regimen. Acne nodules or legions are usually polymorphic, taking many forms, including open or closed comedones, papules, pustules, or cysts, among others.

Uremia refers to high levels of urea in the blood. Urea is one of the primary components of urine. It can be defined as an excess in the blood of amino acid and protein metabolism end products, such as urea and creatinine, which would be normally excreted in the urine. Uremia is generally caused by decreased renal function or renal failure. Symptoms of uremia include skin dryness, itchiness, calciphylaxis, and uremic frost, among others.

Non-contact dermatitis refers to skin conditions that do not involve direct contact with a substance. Examples of non-contact dermatitis include cradle cap, atopic dermatitis, dermatitis herpetiformis, and other forms of inflammatory skin disorders that do not involve direct contact with a substance.

The application 125 can include, present, or otherwise provide a user interface 130 including the one or more UI elements 135 to a user of the user device 110 in accordance with a configuration on the application 125. The UI elements 135 may correspond to visual components of the user interface 130, such as a command button, a text box, a check box, a radio button, a menu item, and a slider, among others. In some embodiments, the application 125 may be a digital therapeutics application and may provide a session (sometimes referred to herein as a therapy session) via the user interface 130 addressing for a propensity for maladaptive scratching.

The application 125 can receive an instruction for presentation of a task 170 to the user. The task 170 can be or include a stimulus or action to be presented textually, as an image, as a video, auditorily, or other presentation to the user and can include instructions for the user to perform the action to reduce or mitigate the desire or propensity to participate in the maladaptive behavior. The task 170 can include interacting or not interacting with the user device 110. For example, the task 170 can include tapping the user interface 130 or not tapping the user interface 130 for a duration of time. The task 170 can include instructions for the user to reduce severity of her condition. For example, the message can include instructions which aim to increase the user's calmness or mental stamina. The task 170 can include an interactive interface, through the user interface 130, to engage the user in one or more therapies designed to reduce the propensity to participate in the maladaptive behavior. For example, the user may play a game on the user device 110 presented by the application 125 which incorporates one or more therapies to address the behavior.

Therapies to reduce the propensity for behavior associated with the skin pathology may include inhibitory control training, habit reversal therapy, or an urge surfing paradigm. Inhibitory control training can include tasks such as a GNG task, in which the user is prompted by the task 170 to perform an action for a period of time, and then to refrain from performing that action for a period of time. HRT can refer to a therapy in which the user is instructed to replace the maladaptive behavior with a permissible behavior. In some implementations, the permissible behavior may be physically incompatible with the maladaptive behavior, thereby preventing the user from participating in the maladaptive behavior while performing the permissible behavior. Urge surfing may refer to a therapy to distract or aid the user in persevering through resisting the propensity to participate in the maladaptive behavior, such as through the application 125 operating on the user device 110.

The database 160 may store and maintain various resources and data associated with the session management service 105 and the application 125. The database 160 may include a database management system (DBMS) to arrange and organize the data maintained thereon. The database 160 may be in communication with the session management service 105 and the one or more user devices 110 via the network 115. While running various operations, the session management service 105 and the application 125 may access the database 160 to retrieve identified data therefrom. The session management service 105 and the application 125 may also write data onto the database 160 from running such operations.

Such operations may include the maintenance of the user profile 165 (sometimes herein referred to as a subject profile). The user profile 165 can include information pertaining to a condition of a user, as described herein. For example, the user profile 165 may include information related to the severity of the condition, occurrences of the condition (such as itchiness caused by the skin pathology), medications or treatments the user takes for the condition, a duration of the condition, among others. The user profile 165 can be updated responsive to a schedule, periodically, (e.g., daily, weekly), a change in user information (e.g., input by the user via the user interface 130 or learned from the user device 110), or a clinician (e.g., a doctor or nurse) addressing the user's condition, among others.

The user profile 165 can store and maintain information related to a user of the application 125 through user device 110. Each user profile 165 may be associated with or correspond to a respective subject or user of the application 125. The user profile 165 may contain or store information for each session performed by the user. The information for a session may include various parameters or tasks 170 of previous sessions performed by the user, and may initially be null. The user profile 165 can enable streamlined communications to the user by presenting a task to the user which, based on the user profile 165 at least, is most likely to aid the user in addressing her condition or reducing the propensity to participate in the maladaptive behavior. This directed approach can reduce the need for multiple communications with the user, thereby reducing bandwidth and increasing the benefit of the user-computer interaction.

In some embodiments, the user profile 165 may identify or include information on a treatment regimen undertaken by the user, such as a type of treatment (e.g., therapy, pharmaceutical, or psychotherapy), duration (e.g., days, weeks, or years), and frequency (e.g., daily, weekly, quarterly, annually), among others. The user profile 165 may include data collected by the sensors 120, such as a heartrate, breathing pattern, or pupil dilation of the user. The user profile 165 may be stored and maintained in the database 160 using one or more files (e.g., extensible markup language (XML), comma-separated values (CSV) delimited text files, or a structured query language (SQL) file). The user profile 165 may be iteratively updated as the user provides responses and performs the tasks 170. For example, the performance evaluator 155 may update the user profile 165 based on a completion of a task 170 by the user.

In addition, identifications of the tasks 170 may be stored and maintained on the database 160. For example, the database 160 may maintain tasks 170 using one or more data structures or files (e.g., extensible markup language (XML), comma-separated values (CSV) delimited text files, or a structured query language (SQL) file). Each of the tasks 170 may prompt the user via the application 125 to perform an action 220 via the application 125. For example, the application 125 may receive instructions to present an activity to be performed as a part of the task 170. The tasks 170 may be to provide behavioral therapies, including cognitive behavioral therapy, habit reversal therapy, biofeedback, and relaxation therapy that may also be helpful for reducing the propensity to participate in the maladaptive behavior. The tasks 170 may be presented as games, activities, or actions to be performed by the user via the user interface 130. For example, the tasks 170 may prompt the user to make a fist with her hand, to interact with the user interface 130, or instruct the user to abstain from an action performed via the user interface 130 or her own body.

In response to the presentation of an indication prompting the user to perform the specified task 170, the user may use the application 125 to record the performance or completion of the task 170. Examples of the tasks 170 may include a breathing exercise, a mind-body exercise, or hand exercise, among others. The tasks 170 may include a GNG task, and HRT task, an urge surfing task, or a combination thereof, among others. The task 170 can be text-based, an image, or a video, among other presentation methods. The task 170 can be presented to the user via the user interface 130 running on the application 125. Each task 170 may identify or include information to be presented via the user interface 130 of the application 125. For example, the information of the task 170 may include reminders to perform the task 170 of the session or to perform an action to mitigate the behavior. The task 170 may be delivered periodically, such as daily, weekly, or monthly, among others. The task 170 may be delivered according to a schedule, or responsive to reception of the indication from the user. The task 170 may be derived from a library of pre-generated psychotherapy activities or actions and/or a library of pre-generated engagement messages. The task 170 may include reminders for the subject to take medication, to practice an activity, or to perform other actions which may reduce the propensity to maladaptively scratch, among other behaviors. The task 170 may be personalized based on the user's activity, adherence, or performance in relation to the regimen. For example, the task 170 may be personalized based on information in the user profile 165, or based on other tasks 170 the user has participated in.

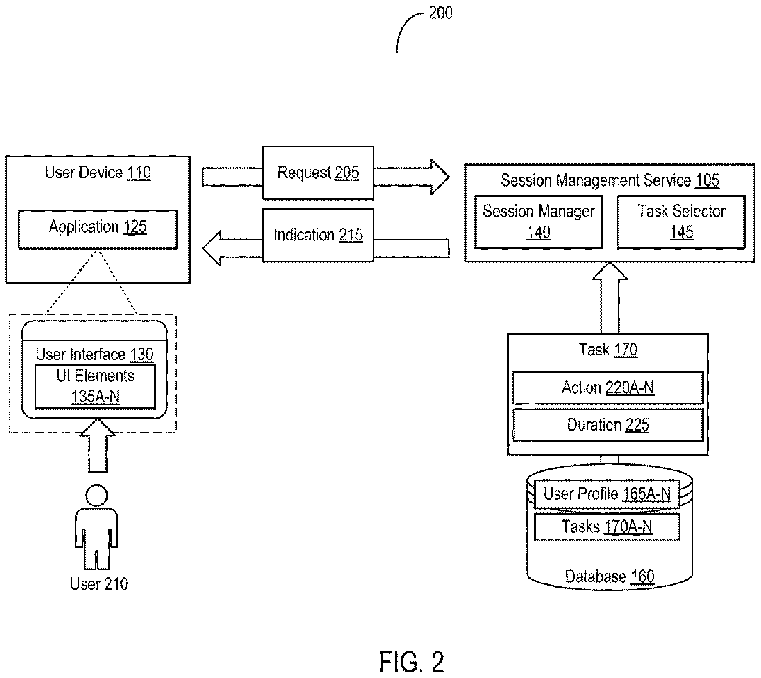

Referring now to FIG. 2, depicted is a block diagram for a process 200 to identify a session, receive a request indicating the onset of the behavior and to provide an indication to perform a task in the system 100 for providing training to address maladaptive behavior in users. The process 200 may include or correspond to operations performed in the system 100 to present user interfaces to users with skin pathologies. Under the process 200, the session manager 140 executing on the session management service 105 may access the database 160 to retrieve, fetch, or otherwise identify the user profile 165 for a user 210 (sometimes herein referred to as a subject, patient, or person) of the application 125 on the user device 110. The user profile 165 may identify or define information associated with the user 210, the instance of the application 125 on the user device 110, and the user device 110, among others. For example, the user profile 165 may identify that user 210 has a certain propensity to participate in maladaptive scratching. The user profile 165 may identify taking of medication by the user 210 to address the skin pathology or other condition and a frequency of maladaptive behavior in the user 210, among others.

With the identification of the user profile 165, the session manager 140 may determine or identify a session for the user 210 for addressing the propensity for the behavior. The session may correspond to, include, or define a set of tasks 170 to be performed by the user 210 via the application 125. Each task 170 may be performed by the user 210 using at least one hand of the user 210. The task 170 may define, specify, or otherwise identify at least an action 220A-N (hereinafter action 220) and at least one duration 225 for each action 220. The action 220 may be, for example, to clench a hand of the user 210, to use the hand to tap or otherwise interact with the user interface 130 via the UI elements 135, to make tapping motions with the hand of the user 210, among other actions involving one or more hands of the user 210. The actions 220 may involve activities without the use of the user's hands. For example, the action 220 may include focusing on a point on a screen of the user device 110, practicing a breathing exercise, or tapping a foot of the user 210, among others. Each task 170 may also have a set duration 225 of time, ranging anywhere from between a few seconds (e.g., 40 seconds) to minutes (e.g., 3 minutes) to a few hours (e.g., 3 hours). Each task may include one or more actions 220 to be performed as a part of that task.

The tasks 170 may specify different sets of actions 220 for various types of tasks. Each task 170 may correspond to a different therapy, such as HRT, GNG, or urge surfing, among others. The task 170 may include one or more actions corresponding to the therapy associated with the task 170. For example, when the task 170 is a GNG task, the actions 220 may include a first action 220A for the user to press a screen of the user interface 130 for a first duration 225. The first duration 225 may be any range of time for a GNG task, such as 5-10 minutes. In this example, the user may be prompted by the application 125 to press the screen of the user interface 130 for 7 minutes, and then the application 125 may present a second action 225B. The second action 225B may include prompting the user to cease pressing the screen for a second duration 225.

Continuing on, when the task 170 is an HRT task, the actions 220 may include prompting the user to understand that resisting an urge (e.g., the maladaptive behavior of scratching) may lead to the urge dissipating and directing the user to perform a permissible behavior different from the maladaptive behavior. For example, the first action 220A may alert the user to cues indicating the onset of the maladaptive behavior, such as a tingling sensation of the skin, an increased heartrate, or other indication. The second action 220B of the HRT task 170 may prompt the user to clench a fist, focus on a point on the user interface 130, or pinch the user's fingers together for a duration 225. In some embodiments, the duration 225 may be between 2-3 minutes for an HRT task. When the task 170 is an urge surfing task, the task 170 may include actions related to managing the propensity to perform the maladaptive behavior and withstanding discomfort resulting from resisting the propensity. A first action 220A of an urge surfing task may be to play a game associated with the application 125, to watch a video associated with the application, or controlling the application based on information detected by the sensors 120 for the duration 225 of the propensity for the maladaptive behavior. The duration 225 may be between 2-5 minutes for an urge surfing task.

The user profile 165 may include information on prior sessions, such as previous tasks 170 performed by the user 210 in response to previous indications 215, requests 205 by the user 210 to initiate a session, a performance associated with tasks 170 already performed by the user 210, taking of medication by the user 210 to address the condition of the user, or information detected by the sensors 120, among others. The user profile 165 may also identify or include information on recorded performance of the maladaptive behavior, such as a number of occurrences of the propensity to perform the maladaptive behavior, a number of occurrences of performing the maladaptive behavior, durations of prior occurrences, and taking of medication, among others. The user profile 165 may initially lack information about prior sessions, and may build information as the user 210 performs the tasks 170 via the application 125. The user profile 165 can be used to select the task 170 to provide via the application 125 to the user 210 in the session.

The session manager 140 may initiate the session responsive to receiving a request 205 from the user 210 via the application 125. The user 210 may provide, via the user interface 130 to execute through the application 125, the request 205 to start a session. The request 205 may include information related to the onset of the user's condition. The request 205 can include attributes associated with the condition such as an identification of the user 210 or the user profile 165, symptoms associated with the condition of the user 210, a time of the request 205, a severity of the propensity, among others. The application 125 operating on the user device 110 can generate the request 205 to send to the session manager service 105, in response to an interaction by the user 210 with the application 125. In some embodiments, the session manager 140 may initiate the session responsive to a scheduled session time, a detection by the sensors 120, based on the user 210 taking a prescribed medication to address the condition, among others.

For the session, the task selector 145 can identify or select the task 170 to provide to the user 210 via the application 125. The selection of the task 170 based on the completion of a prior task 170, based on information in the user profile 165, or based on the severity of the propensity to perform the maladaptive behavior, among others. In some embodiments, the session manager 140 may select a set of tasks 170 to provide to the user 210. For example, the session manager 140 may select a first task (e.g., go/no-go), a second task (e.g., HRT), and then a third task (e.g., urge surfing). The task selector 145 may select the task 170 based on the user profile 165. The user profile 165 may include historical information related to the user's condition, such as occurrences or types of symptoms, time of symptom occurrences, the intensity of the propensity to perform the maladaptive behavior, demographic information, prescription information, location information, among others. For example, the task selector 145 may select a task 170 which has historically improved the user's propensity to perform the maladaptive behavior.

In some embodiments, the task selector 145 may select the task 170 based on a predefined schedule of tasks. For example, the task selector 145 may select the task 170 to be a GNG therapy task in accordance with the predefined schedule of tasks. In this illustrative example, the task selector 145 may select a subsequent task based on the subsequent task of the predefined schedule. For example, a predefined schedule of tasks 170 may include a first task corresponding to a GNG therapy, a second task corresponding to an HRT therapy, or a third task corresponding to an urge surfing therapy. In some embodiments, the task selector 145 may select the task 170 based on a set of rules. The rules may be configured to provide a task 170 or set of tasks 170 to target the underlying causes or alleviate the symptoms of the condition or skin pathology in the user 210 in a systematic, objective, and therapeutically effective manner. The rules may be based around time of completion of the task 170, the user profile 165, a performance of the task 170, or other attributes of the system 100.

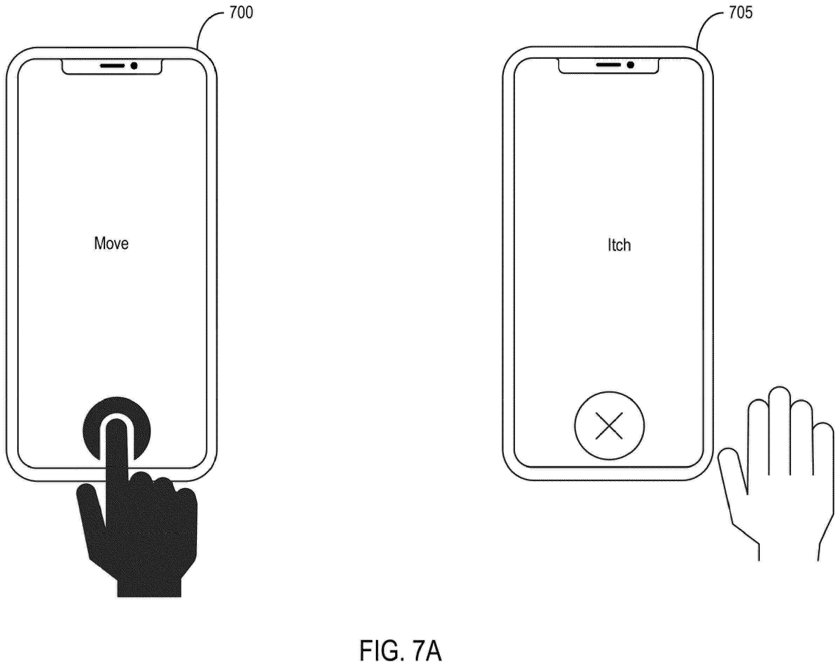

Upon selection, the session manager 140 may provide, send, or otherwise transmit the task 170 or the indication 215 to the user device 110. In some embodiments, the session manager 140 may send an instruction for presentation of the task 170 via the user interface 130 for the application 125 on the user device 110. The instruction may include, for example, a specification as to which UI elements 135 are to be used and may identify content to be displayed on the UI elements 135 of the user interface 130. The instructions can further identify or include the task 170. The instructions may be code, data packets, or a control to present the task 170 to the user 210 via the application 125 running on the user device 110. The instructions may include processing instructions for display of the task 170 on the application 125. The instructions may also include instructions for the user 210 to perform in relation to their session. For example, the instructions may display a message instructing the user to take a medication associated with their session. The instructions may include the task 170 identifying or including at least one stimulus associated with the condition. The stimulus may include a text, image, or audio presented by the user device 110 via the application 125. For example, the stimulus may include presentation of an image instructing the user 210 to interact with the application 125 via the user interface 130, such as those described in FIGs. 7A-13.

In some embodiments, the session manager 140 may calculate, identify, or otherwise determine a time at which to send or present the indication 215 via the user interface 130 of the application 125. In some embodiments, the session manager 140 may maintain a timer to count the time elapsed since the presentation of a previous indication 215. The timer may count the time elapsed from a read receipt of the indication 215, from a transmittal sent from the application 125 as a part of a transfer protocol, or from the transmittal of the indication 215 from the session management service 105, among others. In some embodiments, the session manager 140 may maintain a timer to count the time elapsed since the performance of a previous task. Using the timer, the session manager 140 may determine whether the elapsed time exceeds a threshold. The threshold may delineate, identify, or define an amount of time at which to provide the indication 215 for presentation. When the elapsed time exceeds a threshold, the session manager 140 may send an indication 215. Conversely, if the time counted by the timer does not exceed the threshold, the session manager 140 may refrain from sending the indication 215 and may continue updating the time.

In some embodiments, the session manager 140 may provide the indication 215 in accordance with a schedule. The schedule may identify or specify that time at which the indication 215 is to be presented or transmitted. For example, the schedule may specify for the presentation of the indication on an hourly, daily, or weekly basis. The schedule may be defined in accordance with a prescription medicine schedule or based on the user's historical data, such as contained in the user profile 165. When the time matches the schedule, the session manager 140 may provide the indication 215 for display on the user interface 130.

Upon receipt of the indication 215, the application 125 on the user device 110 may render, display, or otherwise present the indication 215. The indication 215 may be presented via the one or more UI elements 135 of the user interface 130 of the application 125 om the user device 110. The presentation of the UI elements 135 can be in accordance with the task 170 provided to the user 210 via the application 125. The user 210 may perform the task 170 by interacting with the application 125 executing on the user device 110. The user 210 may perform one or more actions 220 of the task 170 for a duration 225. Performing the task 170 can include performing an interaction or a non-interaction with the stimulus. An interaction with the stimulus may include speaking or making another noise to the user device 110, pressing or tapping on one or more of the UI elements 135, rotating or otherwise manipulating the user device 110, among others. A non-interaction with the stimulus may include not tapping or pressing on the UI 130 or a portion of the UI 130 or holding the user device 110 in a specified position. If the user 210 does not complete the task, the session manager 140 may send a subsequent indication 215 to prompt the user 210 to complete the task, or the session manager 140 may abort the task 170 upon the passage of a threshold period. With completion of the task 170, the application 125 may transmit a completion of the task 170 from the user device 110.

In some embodiments, the application 125 can render, display, or otherwise present the indication 215, independently of the session management service 105. The application 125 may share or have the same functionalities as the session manager 140 as discussed above. For example, the application 125 may maintain a timer to keep track of time elapsed since presentation of a previous indication 215. The application 125 may compare the elapsed time with a time limit for the indication 215. When the elapsed time exceeds the time limit, the application 125 may determine to present the indication 215. The application 125 may also use a schedule to determine when to present the indication 215. The application 125 may present the indication 215 for display through the user interface 130 on the user device 110. The indication 215 may include a response mechanism, such as UI elements 135, to provide information related to the condition of the user 210 as well as to indicate an acceptance or denial of the task 170.

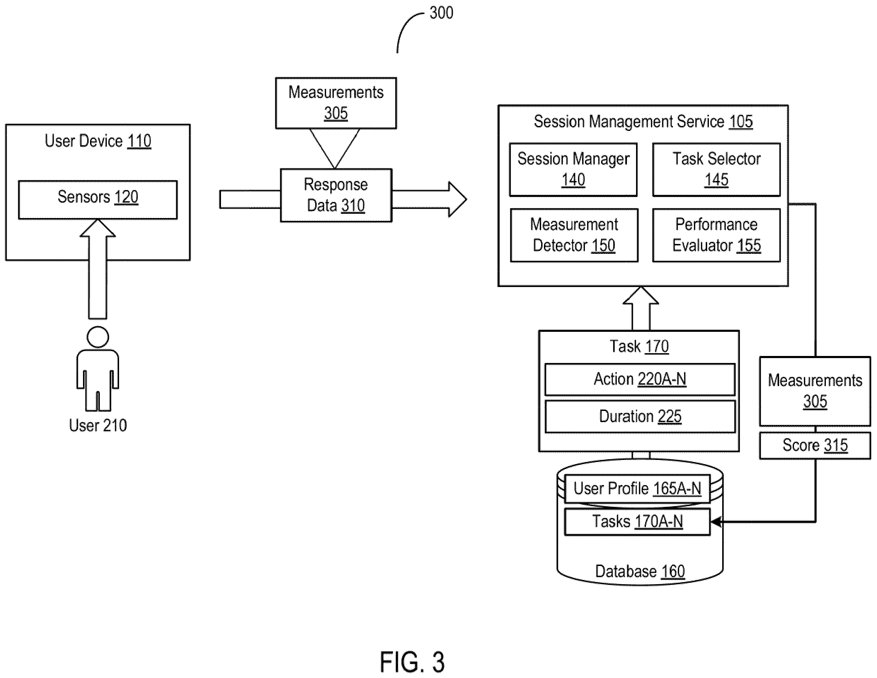

Referring now to FIG. 3, depicted is a block diagram for a process 300 to assess user performance based on response data in the system 100 for providing training to address maladaptive behavior in users. The process 300 may include or correspond to operations to configure visual elements for presentation to address maladaptive behavior in users. Under the process 300, in overview, the measurement detector 150 on the session manager service 105 may retrieve, identify, or otherwise receive the response data 310 from the application 165 running on the user device 110. In some embodiments, the measurement detector 150 may receive the response data 310 periodically, as a result of the elapse of a threshold time, by querying the user device 110 for the response data 310, if the response data 310 is outside of a range of response data for the user profile 165, among others. For example, the measurement detector 150 may receive the response data 310 every minute, hour, or according to a schedule of data transmittal. The measurement detector 150 may query for the response data 310 after the elapse of a threshold time. For example, the measurement detector 150 may receive the response data 310 if the user performs the task 170 for longer than the threshold time.

The response data 310 may include or identify user interaction by the user 210 with the task 170. The response data 310 may include a time for task completion. For example, the response data 310 may include that the user spent 4 minutes on a first task and 7 minutes on a second task. The response data 310 can include a total time for completion of the session and may also include a time of initiation of the session and a time of completion of the session. The response data 310 may identify UI elements interacted with during the duration 225 of the task 170. For example, the response data 310 may include a listing of buttons, toggles, or other UI elements selected by the user 210 at specified times during the performance of the task 170. The response data 310 may include other information, such as a location of the user 210 while performing the session, such as a geolocation, IP address, GPS location, triangulation by cellular towers, among others. The response data 310 may include measurements 305. The measurements 305 may include measurements of time, location, or user data, among others.

In some embodiments, the response data 310 may be sensory data and may identify the measurements 305 of the user 210. The measurements 305 may be or relate to physiological measurements 305 of the user. The measurements 305 may be collected by sensors 120 of the user device 110. The sensors 120 may include sensors to detect biological or physiological information, as described herein. The physiological measurements 305 may be or include measurements pertaining to the body of the user 210. For example, the physiological measurements 305 can include a breathing pattern (e.g., sequence of the lengths of inhalation and exhalation), pupil dilation (e.g., a change in the diameter of the user's pupil), heartrate (e.g., the beats per minutes or an acceleration of heartrate), blood oxygen levels (e.g., the saturation of oxygen within the user 210), blinking pattern (e.g., a rate of blinking of the user's eyes), a moisture level (e.g., of the user's skin or breath), a skin conductance of the user 210, an activity level of the user 210 (e.g., steps per day or other activity indicators), a weight of the user, a height of the user, a focus level of the user (e.g., how long the user 210 is able to look at the user device 110 or participate in the task 170, or the user's skin conductance), among others. The measurement detector 150 may store these measurements 305 and other response data 310 in the database 160.

With the response data 310, the performance evaluator 155 may calculate, generate, or otherwise evaluate a performance or score 315 for the user 210 based on the measurements 305 or the response data 310. The score 315 may identify a reaction time or a correctness of the user 210 in performing the tasks 170, a degree of compliance of the user 210 with the task 170, or a likelihood of overcoming the propensity for the maladaptive behavior, among others. For example, the performance evaluator 155 may determine, from the response data 310, that the user 210 is not performing one or more actions 220 of the task 170, or that the user 210 is not performing the actions 220 of the task 170 within a threshold time (e.g., the reaction time).

The degree of compliance of the user 210 with the task 170 can be based on a response time of the user 210 to an action 220 of the task 170 exceeding or being under a threshold time. For example, if the user 210 takes too long to complete a task 170, then the user 210 may not be fully participating or compliant with the task 170. The degree of compliance of the user 210 can be based on responses provided by the user via the UI elements 135 while performing the task 170. For example, if the user 210 presses a button presented by the application 125 during the task 170 at an incorrect time, or at a time not indicated for pressing the button, the user 210 may have a low degree of compliance. Conversely, if the user 210 completes the task 170 within a threshold time period and performs a threshold number of the actions 220 according to the task 170, then the user 210 may have a high degree of compliance.

The performance evaluator 155 can calculate, generate, or otherwise determine the score 315 related to the likelihood of overcoming the propensity for the behavior. The likelihood of overcoming the propensity for the behavior can refer to, include, or be related to a probability that the user 210 will cease to participate in the maladaptive behavior. For example, if the user 210 succeeds in resisting the propensity each time the propensity arises, the user 210 can be said to have a 100% rate of overcoming the propensity for the behavior. The likelihood of overcoming the propensity for the behavior may include a threshold number of occurrences of the propensity. For example, the performance evaluator 155 may not determine the likelihood until a threshold occurrences of the propensity have arisen, or until a threshold number of tasks 170 have been performed by the user 210. The performance evaluator 155 may determine the likelihood of overcoming the propensity for the behavior based at least on selections of the UI elements 135 during the session, the user profile 165, a time of the session, a time of the tasks 170, a time of the actions 220, among others. In this example, the performance evaluator 155 can generate a score related to the performance of the user 210 on at least one of the tasks 170.

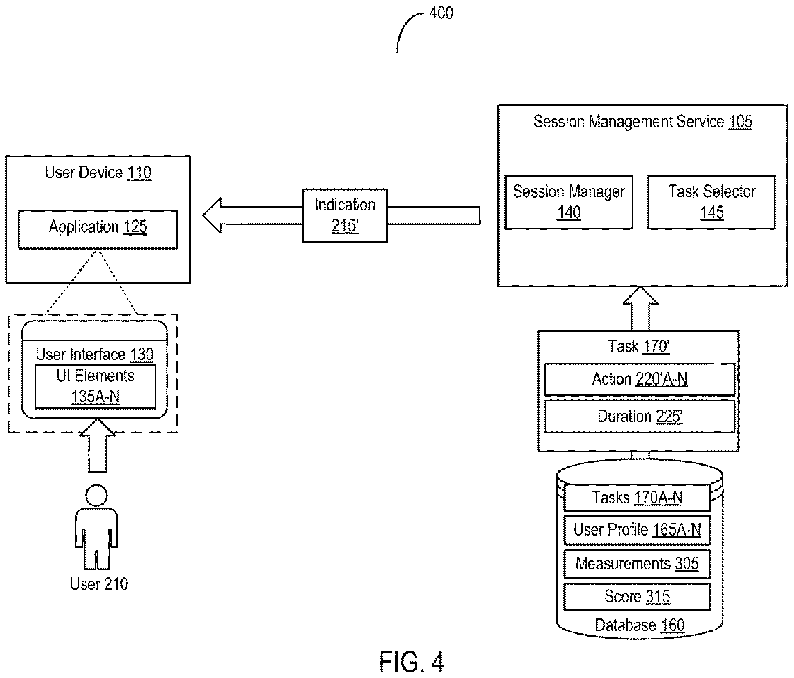

Referring now to FIG. 4, depicted is a block diagram for a process 400 to provide feedback to the user and to determine a task based on a performance of one or more tasks in the system 100 for providing training to address maladaptive behavior in users. The process 400 may include or correspond to operations performed in the processes 100, 200, or 300. Under the process 400, the task selector 145 may identify or select tasks 170' to select for the session manager 140 to provide via the application 125. In some embodiments, the tasks 170' may be for a portion of the session subsequent to the portion in which the task 170 was performed. For example, the task 170' may be performed in a second or third portion of the session, while the task 170 was performed in the first portion of the session. In some embodiments, the tasks 170' may be for another session separate from the session in which the task 170 was performed. The selection of the task 170' may be based on the prior tasks 170, the user profile 165, the measurements 305, the score 315, or the request 205. The task selector 145 may select a subsequent task 170' of the tasks 170 depending on the performance of the user 210 in the preceding task 170. For example, the task selector 145 may select an urge surfing task 170' over a GNGtask if the measurements 305 of the user indicate an increased heartrate.

In some embodiments, the task selector 145 may modify the selection of the task 170' based on the measurements 305. The measurements 305 may indicate a physical state, emotional state, or other state of the user 210 related to the physiology of the user 210. For example, an increased heartrate of the user 210 may indicate that the user 210 is feeling anxious or stressed in relation to an urge to scratch an affected skin. In this example, the task selector 145 can select the task 170' corresponding to the urge surfing task to address the user's anxiety. Based on these physiological measurements 305, the task selector 145 may select the task 170'. In some embodiments, the task selector 145 may modify parameters of the task 170' according to the physiological measurements 305.

In some embodiments, the task selector 145 may identify or select the task 170' based on the time measurements of the measurements 305. In some embodiments, the task selector 145 may select a task 170' based on a measured reaction time during the task 170. For example, the task selector 145 may select a task 170' with a longer duration 225' to allow for a longer reaction time measured during the performance of the task 170. The task selector 145 may modify actions 220', the duration 225', or other parameters of the task 170' based on the measurements 305. For example, the task selector 145 may select actions 220' for the task 170' corresponding to a shorter duration 225' if the measurements 305 indicate that the user 210 has an increased propensity to participate in the maladaptive behavior and if the measurements 305 indicate that the user 210 performed the prior task 170 below a threshold time.

The task selector 145 can identify or select the task 170' based on the score 315. In some embodiments, the task selector 145 may select the task 170' based on the score 315 corresponding to a reaction time or a correctness. For example, the task selector 145 may select the task 170' to have a longer duration 225' than the task 170 if the score corresponding to a reaction time is below a threshold level. The task selector 145 may select the task 170' to include a higher number of or a lower number of actions 220' if the score corresponding to the correctness of the prior task 170 is below a threshold value. In some embodiments, the task selector 145 may select the task 170' based on the reaction time and the correctness of the prior or any prior tasks.

The task selector 145 may select the task 170' based on the degree of compliance of the user 210. For example, the task selector 145 may select the task 170' corresponding to the user's compliance of a prior task 170. The task selector 145 may select a task of a different therapy type than the prior task 170 if the user 210 has a low degree of compliance for the task 170. Conversely, the task selector 145 may select the same type of therapy for the task 170' if the user 210 exhibited a low degree of compliance for that type of therapy in the prior task 170. The task selector 145 may use any combination of the tasks 170, user profile 165, measurements 305, or score 315 to determine to select or modify a task 170'. For example, the task selector 145 may use the heart rate of the user 210 in conjunction with a correctness score 315 of the user to determine which task of the tasks 170 to provide to the user 210 via the application 125 operating on the user device 110.

In some embodiments, the task selection can select the task 170' in response to a completion of the task 170. For example, the session manager 140 may retrieve, identify, or otherwise receive a completion of the task 170 from the user device 110. Upon receipt, the session manager 140 may process or parse the completion to determine, extract, or identify information related to the user 210, the propensity to perform the maladaptive behavior, the task 170, or other information related to her condition or the task 170. Upon receipt of the completion of a first task 170, the session manager 140 may provide a second task 170'.

With the selection of the task 170', the session manager 140 can send, provide, or otherwise transmit at least one indication 215'. The transmission of the indication 215' can be similar to the transmission of the indication 215 as discussed in process 200. The indication 215' can be an instruction for presentation of the task 170 via the user interface 130 for the application 125 on the user device 110. The instruction may include, for example, a specification as to which UI elements 135 are to be used and may identify content to be displayed on the UI elements 135 of the user interface 130. The instructions can further identify or include the task 170'. The instructions may be code, data packets, or a control to present the task 170 to the user 210 via the application 125 running on the user device 110. The instructions may include the task 170 identifying or including at least one stimulus associated with the condition. The stimulus may include a text, image, or audio presented by the user device 110 via the application 125. For example, the stimulus may include presentation of an image instructing the user 210 to interact with the application 125 via the user interface 130, such as those described in FIGs. 7A-13.

Upon receipt of the indication 215', the application 125 on the user device 110 may render, display, or otherwise present the indication 215'. The indication 215' may be presented via the one or more UI elements 135 of the user interface 130 of the application 125 on the user device 110. The presentation of the UI elements 135 can be in accordance with the task 170' provided to the user 210 via the application 125. The user 210 may perform the task 170' by interacting with the application 125 executing on the user device 110. The user 210 may perform one or more actions 220 of the task 170' for a duration 225. Performing the task 170' can include performing an interaction or a non-interaction with the stimulus. An interaction with the stimulus may include speaking or making another noise to the user device 110, pressing or tapping on one or more of the UI elements 135, rotating or otherwise manipulating the user device 110, among others. A non-interaction with the stimulus may include not tapping or pressing on the UI 130 or a portion of the UI 130, or holding the user device 110 in a specified position. If the user 210 does not complete the task, the session manager 140 may send a subsequent indication 215' to prompt the user 210 to complete the task, or the session manager 140 may abort the task 170' upon the passage of a threshold period. With completion of the task 170', the application 125 may transmit a completion of the task 170' from the user device 110.

In some embodiments, the session manager 140 may produce, output, or otherwise generate feedback for the user 210 to receive via the user interface 130. The session manager 140 may generate feedback based on at least the performance of the user 210 for a task 170, the user profile 165, the request 205, the measurements 305, or the score 315. The feedback may include text, video, or audio to present to the user 210 via the application 125 displaying through the user interface 130. The feedback may include a presentation of the score 315, a string or phrase, or audio or haptic feedback, among others. The feedback may display a message, such as a motivating message, suggestions to improve performance, a congratulatory message, a consoling message, among others. The feedback may include a presentation of the score 315. The session manager 140 may generate the feedback based on the measurements 305. In some embodiments, the session manager 140 may generate the feedback during the performance of the task 170 by the user 210.

Upon generation, the session manager 140 may send, transmit, or otherwise present the feedback for presentation via the application 125 during the performance of the task 170 or subsequent to the completion of the task 170. For example, the measurements 305 may depict an increased skin conductance of the user 210 during the performance of the task 170, which may indicate increased focus of the user 210 on the task 170. The session manager 140 may then generate feedback corresponding to the increased skin conductance, such as a motivating message to display on the user interface 130. Likewise, the measurements 305 may depict an increased heartrate for the user 210, which may indicate increased stress or anxiety for the user 210. The session manager 140 may then present calming feedback, such as a relaxing message, on the user interface 130.

The session manager 140 may present the score 315 in conjunction with, or separately from, the feedback. For example, the session manager 140 may present the score 315 as a part of the feedback, or may present the score 315 at a time different than presenting the feedback, or may not present the score 315 at all. The session manager 140 may transmit the feedback via the indication 215', or as a separate communication. The session manager 140 may transmit the feedback as a part of the subsequent task 170'. For example, the session manager 140 may include in the indication 215' the next task 170' as well as feedback on the performance of the prior task 170. Through this means, the user 210 can be made aware of her progress in addressing the propensity to participate in the maladaptive behavior.

The session management service 105 may repeat the functionalities described above (e.g., processes 200, 300, and 400) over multiple sessions. The number of sessions may be over a set number of days, weeks, or even years, or may be without a definite end point. By repeatedly selecting tasks 170 based at least on the measurements 305, user profile 165, or the score 315, the user 210 may be able to receive content to help alleviate the propensity to participate in the maladaptive behavior. This may overcome the difficulties faced by the user 210 in accessing such care, even when suffering from a condition which could otherwise inhibit the user from seeking treatment or even physically accessing the user device 110. Furthermore, from carrying out the tasks 170 when presented through the user interface 130 of the application 125, the quality of a human computer interactions (HCI) between the user 210 and the user device 110 may be improved.

Since the tasks 170 are more related to the user's conditions (e.g., skin pathologies), the user 210 may be more likely to participate in such tasks 170 when presented via the user device 110. This may reduce unnecessary consumption of computational resources (e.g., processing and memory) of the service and the user device 110 and lower the usage of the network bandwidth, relative to sending otherwise ineffectual or irrelevant tasks 170. Furthermore, in the context of a digital therapeutics application, the individualized selection of the tasks 170 may result in the delivery of user-specific interventions to improve subject's adherence to the treatment. This may result in not only higher adherence to the therapeutic interventions but also lead to potential improvements to the user's condition or propensity to participate in the maladaptive behavior.

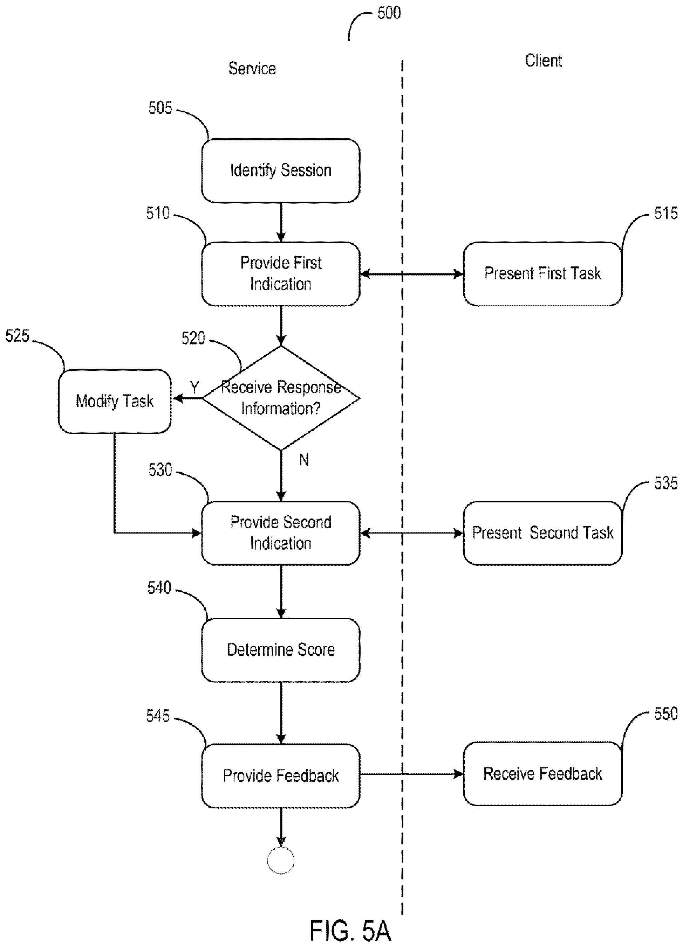

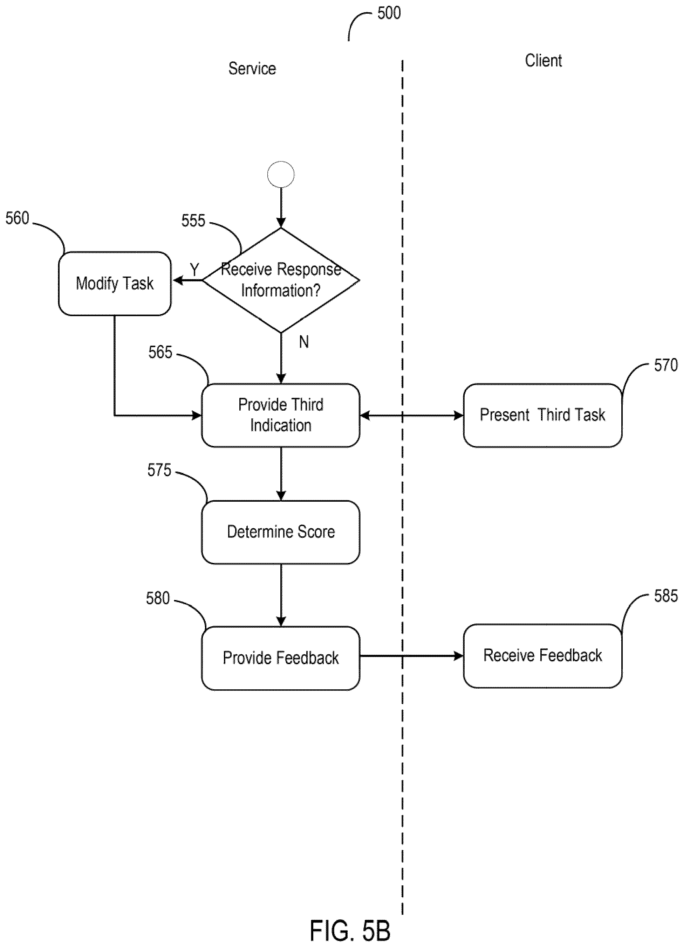

Referring now to FIGs. 5A and 5B, depicted is a method 500 for providing training via visual elements for presentation to address maladaptive behavior in users. Each figure depicts operations performed by the client and operations performed by the service. The method 500 may be implemented or performed using any of the components detailed herein, such as the session management service 105 and the user device 110. Starting from FIG. 5A, under method 500, a service (e.g., the session management service 105) may identify a session (505). The service may provide a first indication (e.g., the indication 215) for a first task (e.g., the task 170) to the client (e.g., the user device 110) (510). The client may present the first indication for a first task (515). Upon completion of the first task, the service may determine whether response information (520). Upon receipt of the response information, the service may modify a second task (e.g., the task 170') (525). The service may provide a second indication (e.g., the indication 215') of the second task (530). The client may perform the second task (535). The service may determine a score related to the performance of one or more of the tasks (540). The service may provide feedback to the client (545). The client may receive the feedback, such as via the user interface (550).

Moving onto FIG. 5B, upon completion of the second task, the service may determine whether response information (555). With receipt of the response information, the service may modify a third task (e.g., the task 170') (560). The service may provide a third indication (e.g., the indication 215') of the third task (565). The client may present the third indication for the third task (570). The service may determine a score related to the performance of one or more of the tasks (575). The service may provide feedback to the client (580). The client may receive the feedback, such as via the user interface (585).

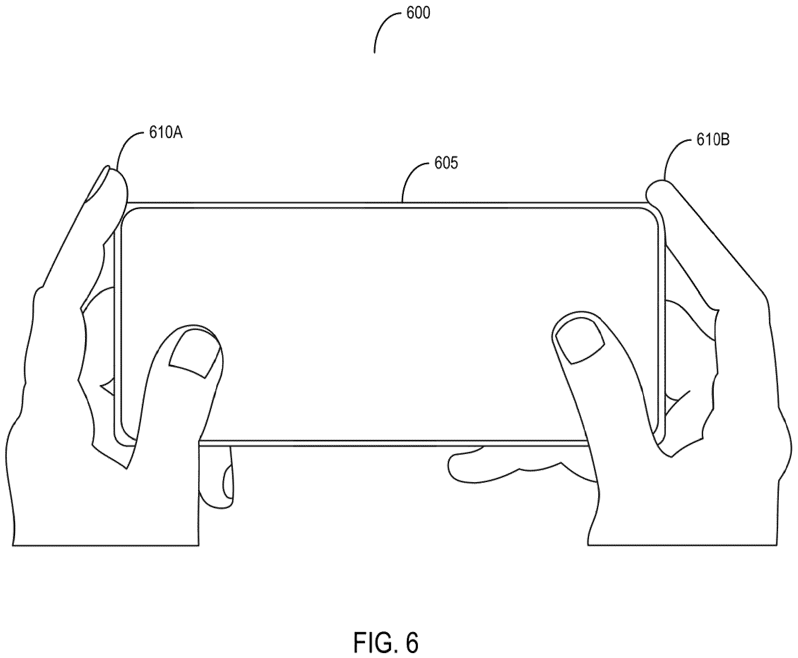

Referring now to FIG. 6, depicted is a sample system 600 presenting a task on a device 605 for a system for providing training via visual elements for presentation to address maladaptive behavior in users. The system can include a client device 605 (e.g., user device 110), a left hand 610A and a right hand 610B. The client device 605 can include the application 125 installed thereon and the sensors 120. In some embodiments, the client device 605 can be similar to the user device 110 and can execute the application 125. The application 125 may present on a user interface or display of the client device 605. In some embodiments, the application 125 may present as a game, image, video, or interactive application 125 on the user device 605. One or two of the hands 610 may be used to interact with the device 605 to perform one or more of the tasks 170. The device 605 may include one or more user elements, such as the UI elements 135, to accept input by the hands 610. The hands 610 may press on a screen of the client device 605 to enter input, may manipulate or rotate the client device 605 to register (via a detection by an accelerometer, gyroscope, or other sensor of the sensors 120) a movement of the client device 605, or the hands 610 may simply hold the client device 605.

Since the application operates on the subject's mobile device, or at least a mobile device that she can access easily and reliably, e.g., according to the predetermined frequency (e.g., once per day), the application provides real-time support to the subject. For example, upon receiving a request from the user to initiate a session, the application initiates in real-time, i.e., within at least a few milliseconds from receiving the request, a session. Such prompt guidance cannot be achieved via in-person visits, phone calls, video conferences or even text messages between the user and health care providers, such as dermatologists. In this manner, the application is able to provide and customize tasks for the user based on the performance of the user. This can create an iteratively improving service for the user wherein overall bandwidth and data communications are minimized due to the increasing usefulness of each task.

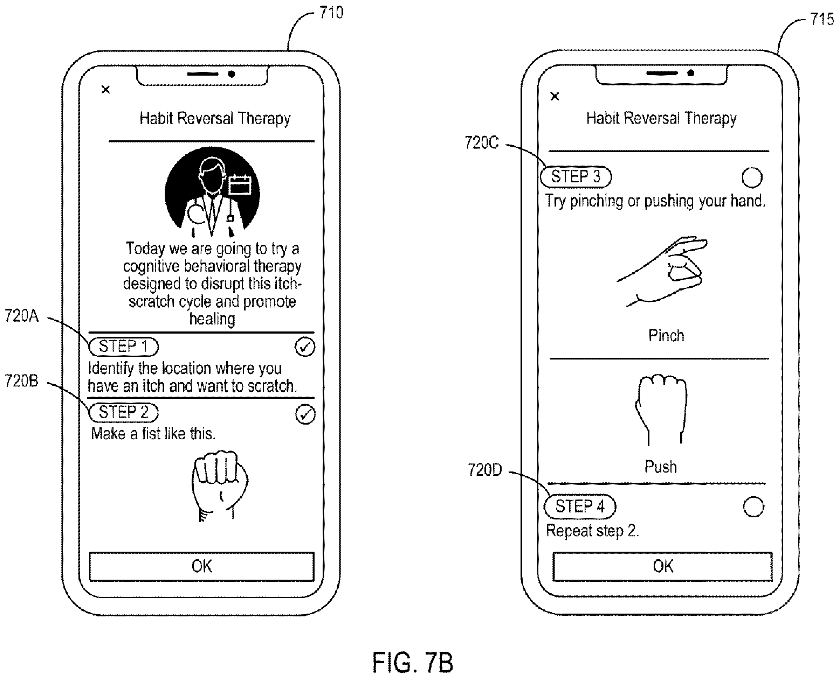

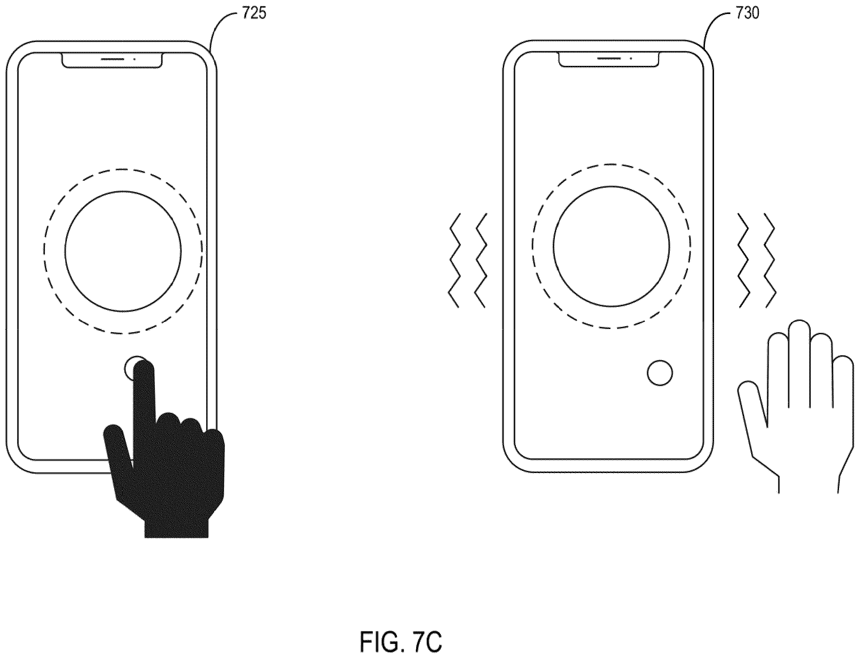

FIGs. 7A-7C depict screenshots of example user interfaces 700-725 for a system for providing training via visual elements for presentation to address maladaptive behavior in users. FIG 7A depicts a GNG therapy task operating through an application on a client device. The client device displays a user interface 700 to "move," (e.g., interact with the client device). The client device displays a user interface 705 to "itch," (e.g., do not interact with the client device). This GNG task motivates the user to interact with the device like in user interface 700 when the word displayed on the screen is not related to the maladaptive behavior. The GNG task then shows the user not interacting with the client device when the user interface 705 displays a word related to the maladaptive behavior. FIG. 7B displays an exemplary HRT task including steps 720. In a first display 710, a first step 720A and a second step 720B are depicted. In a second display 715, a third step 720C and a fourth step 720D are depicted. The steps 720 on the displays 710 and 715 depict an HRT task including a lesson to perform operations with the hand of the user to perform a function physically incompatible with the maladaptive behavior. FIG. 7C depicts a GNG task interface. The display 725 depicts an action of a task to tap a circle as quickly as possible. The display 730 depicts an action of the task to withhold a response when the phone vibrates or provides haptic feedback.

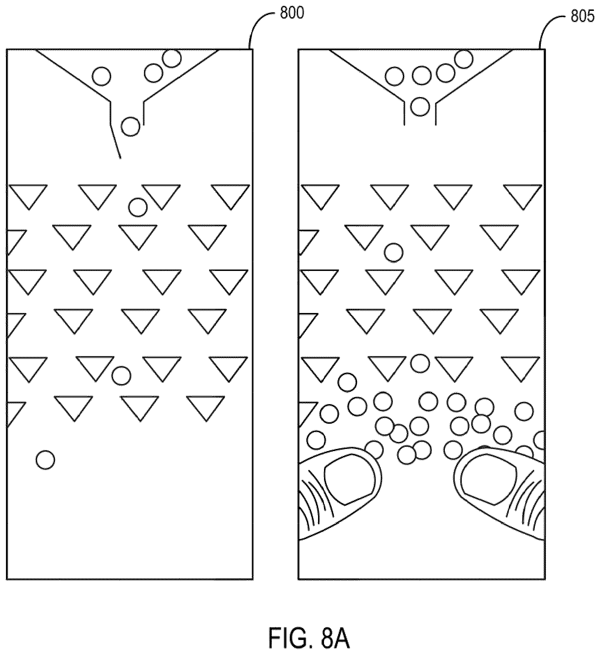

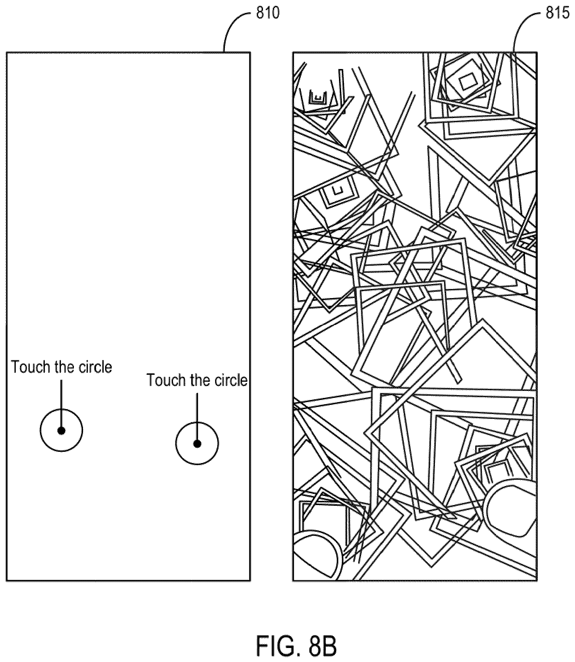

Referring now to FIGs. 8A-8B, depicted are screenshots of a set of example user interfaces (800-815) for a system for providing training via visual elements for presentation to address maladaptive behavior in users. FIG. 8A depicts an urge surfing task. A first user interface 800 depicts a series of balls falling down responsive to the user providing an initial tap on the user interface 800. A second user interface 805 depicts thumbs of the user interacting with the balls on the user interface 805 by pressing the user interface 805. FIG. 8B depicts an urge surfing task including a first user interface 810 and a second user interface 815. The first user interface 810 prompts the user to place her thumbs on the user interface 810. The second user interface 815 displays shapes emanating from the position of the user's thumbs.

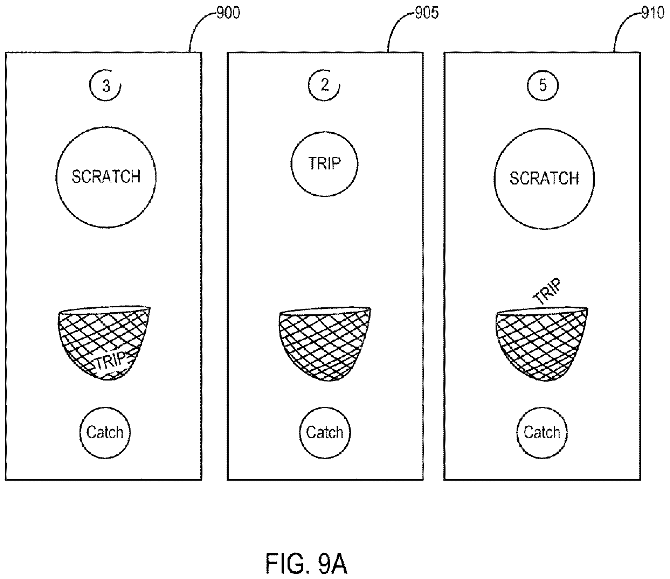

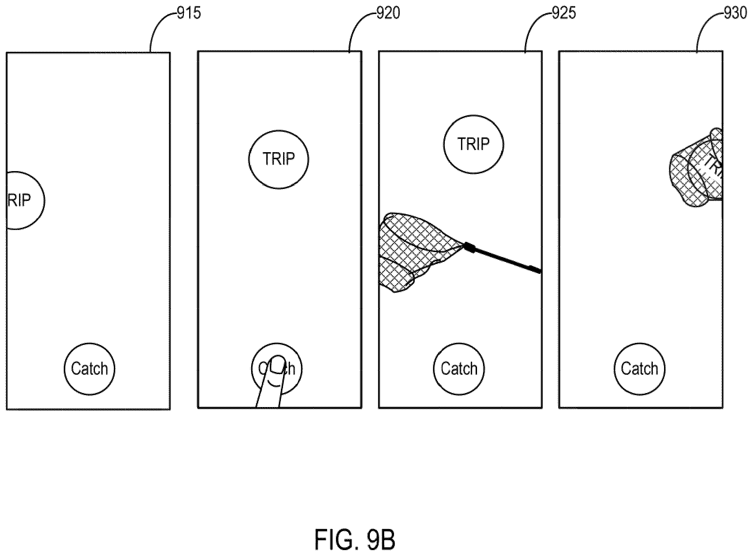

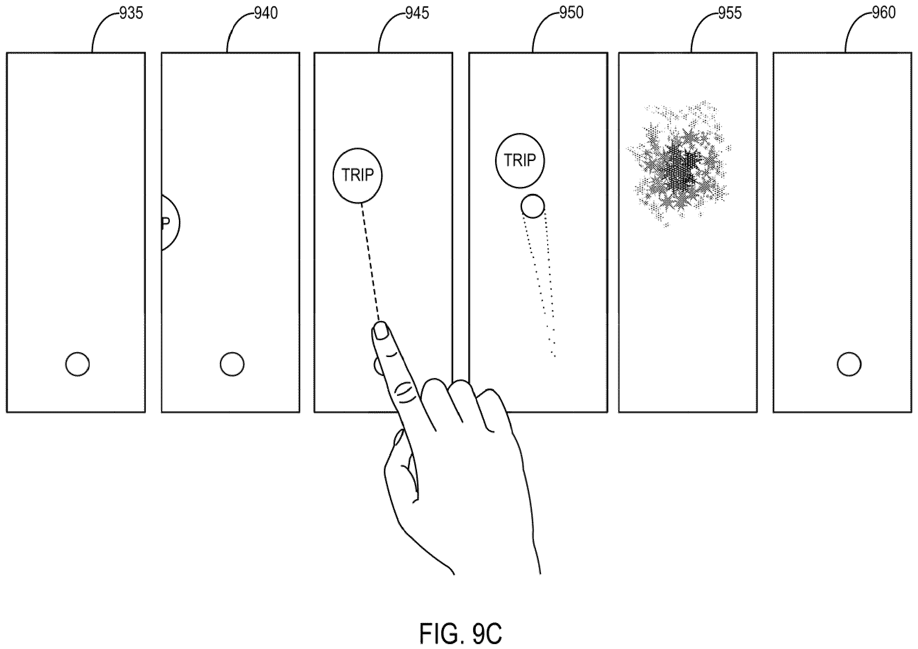

Referring now to FIGs. 9A-9C, depicted are screenshots of a set of example user interfaces (900-945) of a system for providing training via visual elements for presentation to address maladaptive behavior in users. FIG. 9A depicts a GNG task including a first user interface 900, a second user interface 905, and a third user interface 910 for the user to perform in accordance with the GNG task. The first user interface 900 depicts a word associated with the maladaptive behavior and the user is to abstain from interacting with the word. The second user interface 905 depicts a word not associated with the maladaptive behavior, or a word with a positive association. During the second user interface 905, the user may perform an action to catch the positive or neutral word. In the third user interface 910, a word associated with the maladaptive behavior is displayed for the user to ignore. FIG. 9B depicts a GNG task including a first user interface 915, a second user interface 920, a third user interface 925, and a fourth user interface 930. During this GNG task, the user is to catch a positive or neutral word that is not associated with the maladaptive behavior or skin pathology. Conversely, the user is to ignore words associated with the maladaptive behavior or skin pathology. FIG. 9C depicts a GNG task including user interfaces 935-960 in which the user is to interact with, e.g., by "shooting," the neutral or positive word.

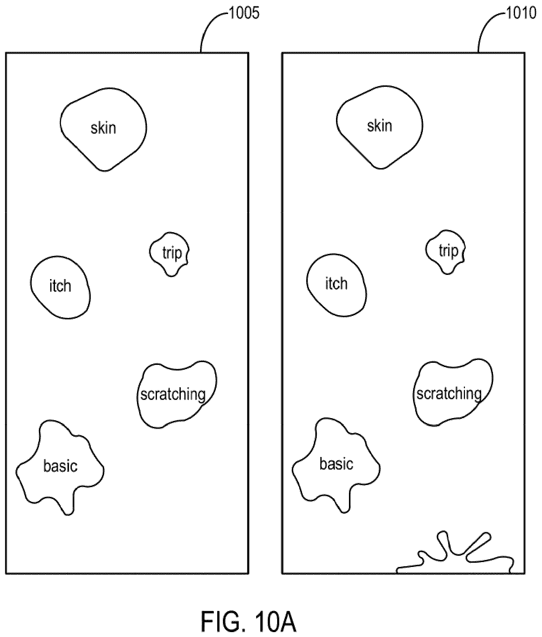

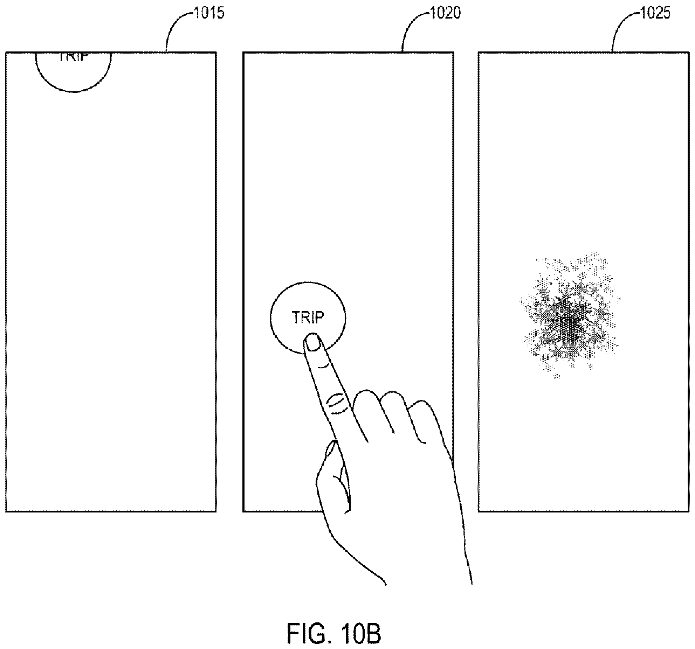

Referring now to FIGs. 10A-10B, depicted are screenshots of example user interfaces (1005-1025) of a system for providing training via visual elements for presentation to address maladaptive behavior in users. FIG. 10A depicts a GNG task including a first user interface 1005 and a second user interface 1010. The GNG task may include the user tapping on the positive or neutral words, and allowing the words associated with the maladaptive behavior or skin pathology to fall to the bottom. FIG. 10B depicts a GNG task including a first user interface 1015, a second user interface 1020, and a third user interface 1025. In this embodiment of GNG task, the user may interact with (e.g., "pop") a bubble containing a positive or neutral word.

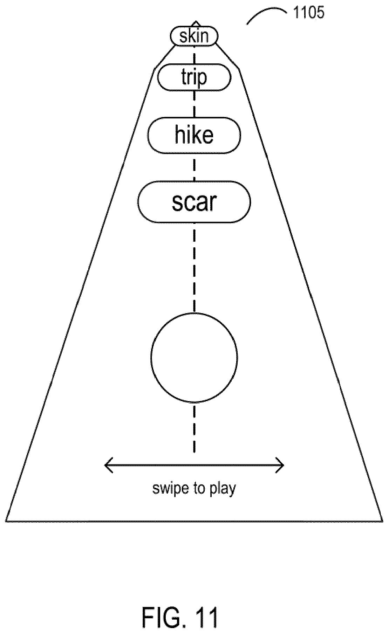

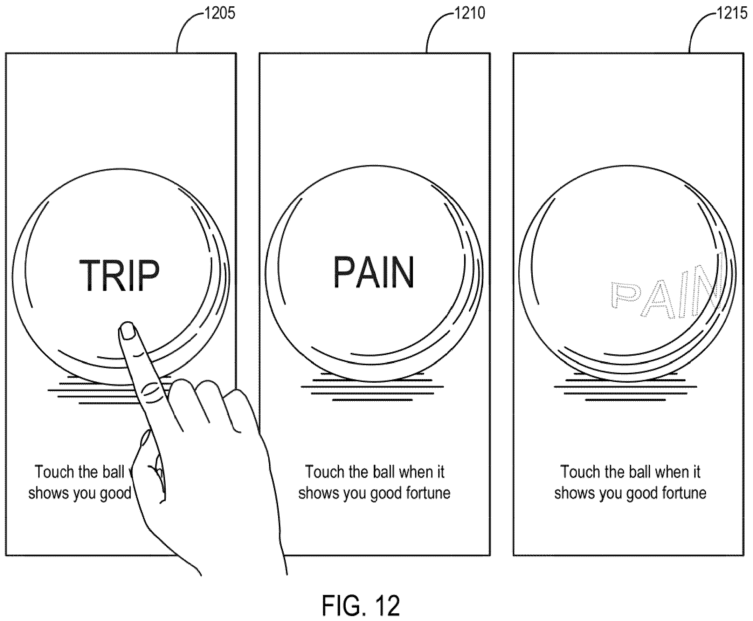

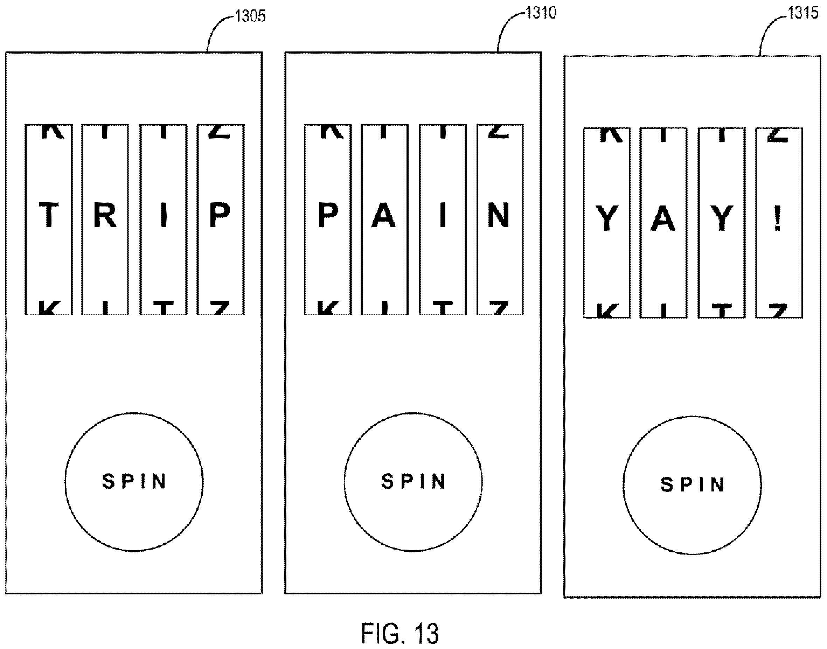

Referring now to FIG 11, depicted is a screenshot of an example task 1105 of a system for providing training via visual elements for presentation to address maladaptive behavior in users. The example task 1105 may be executed by the application 125 via the user device 110. The user may interact with the task 1105 through a user interface, such as the user interface 130. The task 1105 may be a GNG task and may include the user moving a ball to either side of the user interface depending on if a displayed word is associated or neutral with respect to maladaptive behavior or skin pathology. Referring now to FIG 12, depicted is a screenshot of an example task including user interfaces 1205-1215 of a system for providing training via visual elements to address maladaptive behavior in users. The example task may be a GNG task, and may include the user interacting with the user interfaces 1205-1215 by tapping on the screen if a displayed word is positive or neutral. Referring now to FIG 13, depicted is a screenshot of an example task including user interfaces 1305-1315 of a system for providing training via visual elements to address maladaptive behavior in users. The example task may be a GNG task, and may include the user interacting with the user interfaces 1305-1315 if a displayed word is positive or neutral.

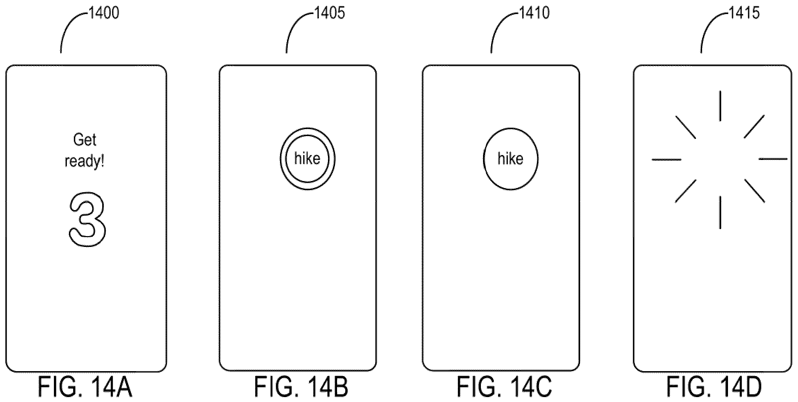

Referring now to FIGs. 14A-D, depicted are screenshots of example user interfaces 1400-1415 of go/no-go training in the system for providing training. The user interface 1400 may be a prompt for the user to be ready for the start of the go/no-go training. The user interface 1405 may follow the user interface 1400, and may direct the user to hold the button with a word that is neutral with respect to the skin pathology (e.g., "hike"). The user interface 1410 may be presented subsequent to the user interface 1405, and may indicate to the user that the go/no-go task is about to be completed. The user interface 1415 may be displayed following the user interface 1410, and may signal the user the completion of the task.

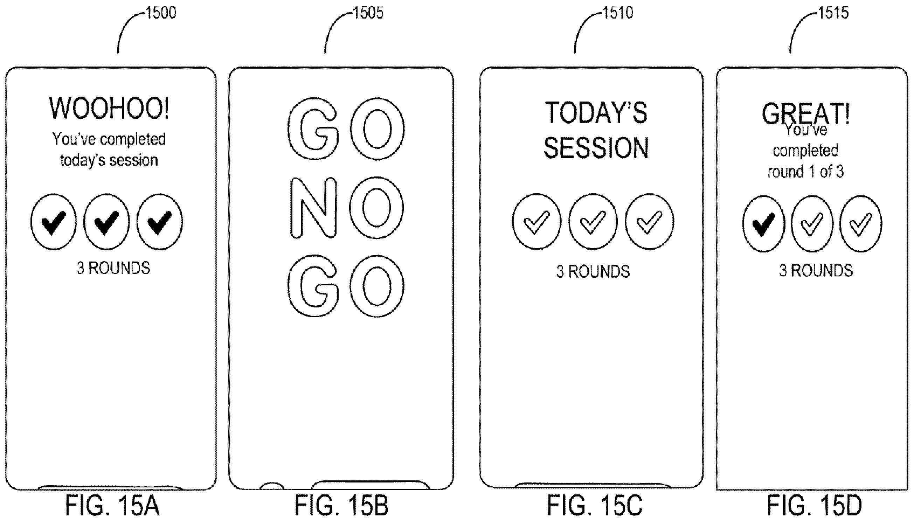

Referring now to FIGs. 15A-D, depicted are screenshots of example user interfaces 1500-1515 of go/no-go training in the system for providing training. The user interface 1500 may indicate all the rounds (or sessions) have been completed by the user in a given day. The user interface 1505 may be a start screen for the go/no-go training task. The user interface 1510 may indicate a total number of available rounds for the user to be performed. The user interface 1515 may indicate that one out of three total number of available rounds have been performed by the user on a given day.

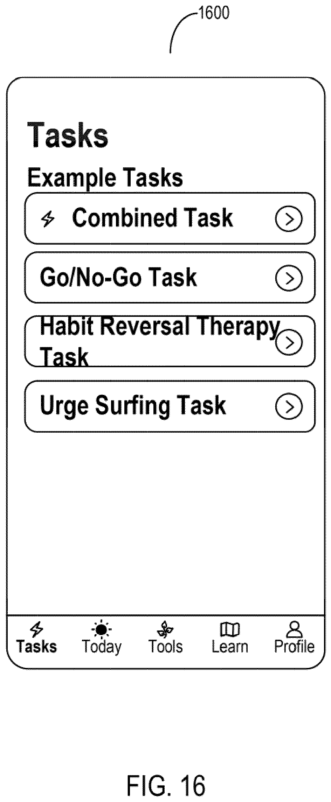

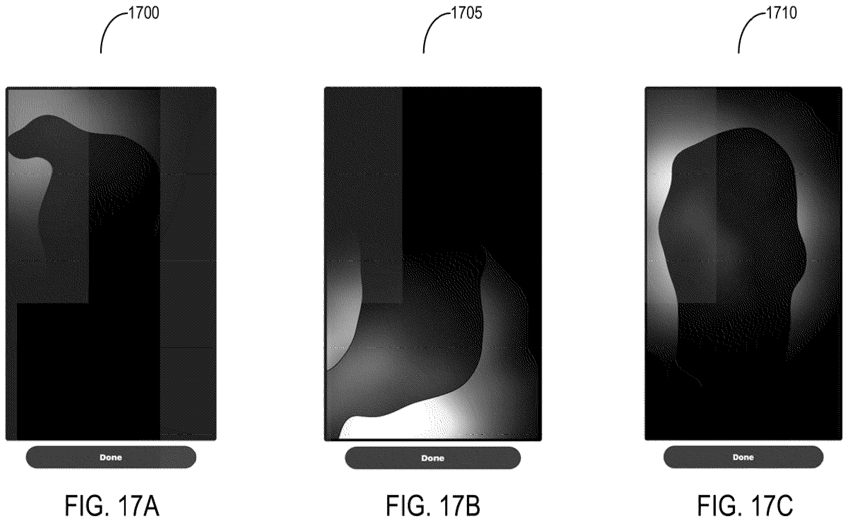

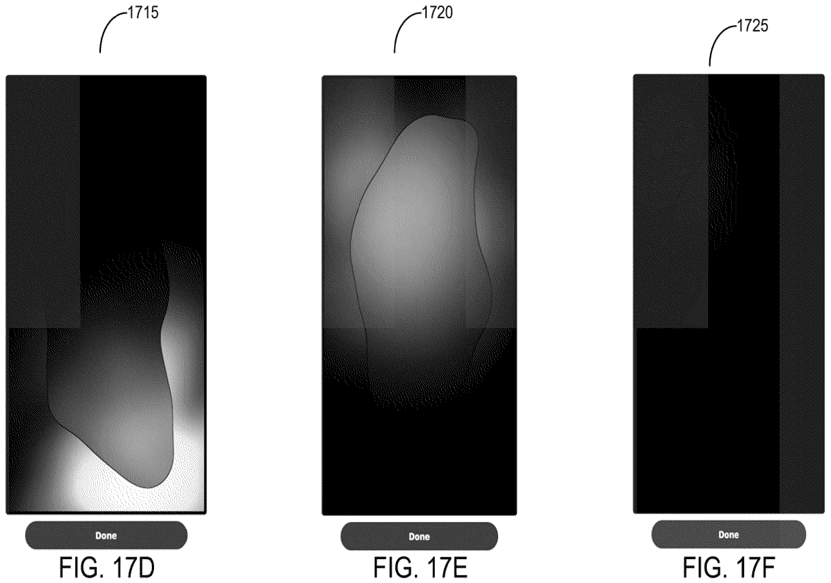

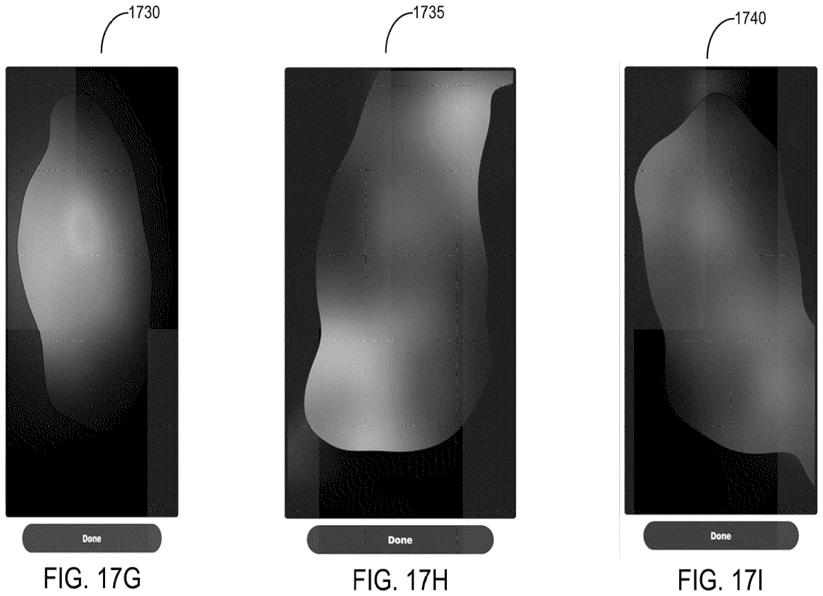

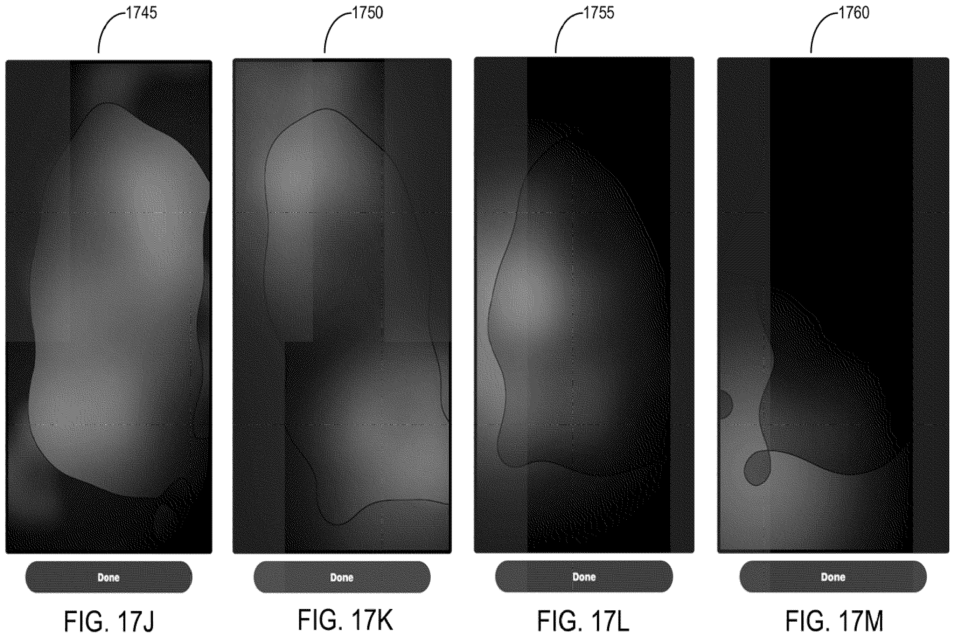

Referring now to FIG. 16, depicted is a screenshot of an example user interface 1600 with options for various interventions in the system for providing training. The user interface 1600 may include a set of user interface elements correspond to available types of training, such as a combined task, a go/no-go task, a habit reversal therapy task, and an urge surfing task, among others. The user of the application may select one of the user interface elements to request for provision of the corresponding task. Referring now to FIGs. 17A-M, depicted are screenshots of example user interfaces 1700-1760 for urge surfing training in the system for providing training. The user interfaces 1700-1760 may be rendered subsequent to selection of the urge surfing training option in the user interface 1600. The user interfaces 1700-1760 may show the morphing of a blob to facilitate the user to visualize and draw attention away from the maladaptive behavior (e.g., scratching) through the duration of the urge to perform such behaviors.

### B. Method of Reducing Frequencies or Intensities for Maladaptive Behaviors Related to Skin Pathologies in Users in Need Thereof

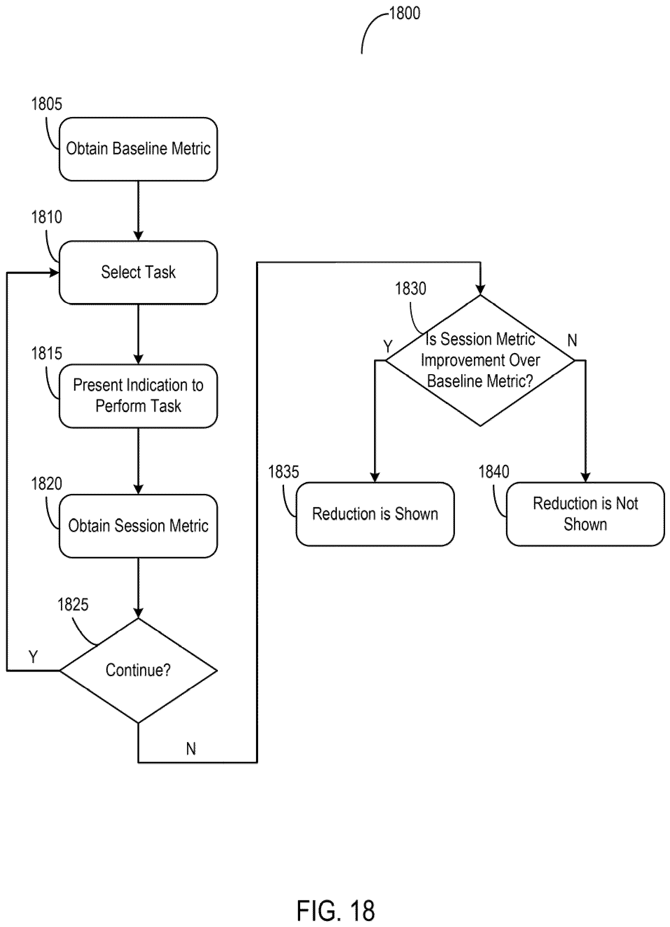

Referring now to FIG. 18, depicted is a flow diagram of a method of reducing a frequency or intensity for a maladaptive behavior related to a skin pathology in a user in need thereof. The method 1800 may be performed by any components or actors described herein, such as the session management service 105, the user device 110, or the user 210, among others. The method 2000 may be used in conjunction with any of the functionalities or actions described herein in Section A or in Section B. In brief overview, the method 1800 may include obtaining a baseline metric (1805). The method 1800 may include selecting a task (1810). The method 1800 may include presenting an indication to perform the task (1815). The method 1800 may include obtaining a session metric (1820). The method 1800 may include determining whether to continue (1825). The method 1800 may include determining whether the session metric is an improvement over the baseline metric (1830). The method 1800 may include determining that a reduction is shown when the session metric is determined to have improved over the baseline metric (1835). The method 1800 may include determining that no reduction is shown when the session metric is determined to have not improved over the baseline metric (1840).

In further detail, the method 1800 may include retrieving, identifying, otherwise obtaining a baseline metric (1805). The baseline metric may be associated with a user (e.g., the user 210) suffering from or diagnosed with a skin pathology. The skin pathology may include one or more of, for example, atopic dermatitis, contact dermatitis, psoriasis, urticaria, prurigo nodularis, lichen planus, acne vulgaris, a drug-induced rash, xerosis, uremia, or non-contact dermatitis (eczema), among others. The skin pathology may be caused by an infection (e.g., from a disease such as chicken pox), allergy (e.g., to plant, animal, medication, or other exposures), or a genetic mutation, among others. The maladaptive behavior related to the skin pathology may include, for example, at least one of continuous scratching of an affected epidermal tissue of the user, an intermittent scratching of the affected epidermal tissue, or a reflexive reaction to a pruritis on the affected epidermal tissue, among others.

The baseline measure may be (e.g., by a computing device) obtained prior to performing any of the time instances or sessions during which a task is to be performed by the user via a digital therapeutics application (e.g., the application 125 or the Study App described herein). The baseline measure may indicate the condition of the user with respect to the severity of the skin pathology and the associated maladaptive behavior on the user's daily life and activities. The baseline metric may include, for example, a dermatology life quality index (DLQI) value, a peak pruritus numerical rating scale (PP-NRS) value, a patient-reported outcomes measurements information systems (PROMIS) value, a patient-oriented eczema measure (POEM) value, a psoriasis symptom inventory (PSI) value, Eczema Area and Severity Index (EASI), digital EASI (EASI-dig), Scoring in Atopic Dermatitis (SCORAD), Patient-Oriented SCORAD (PO-SCORAD), PASI (Psoriasis Area and Severity Index), or Self-Administered PASI (SA-PASI), or a computerized cognitive assessment value, among others. Before performance of any task, the user may have an initial baseline metric satisfying a baseline threshold. The threshold may delineate, define, or identify a value for the initial metric at which the user is identified to be suitable or eligible for performance of the tasks via the digital therapeutics application. The initial metric may be, for example, an itch numerical ration scale (NRS) and the baseline threshold may be a value of 4 or less.

The user may be of any demographic or trait, such as by age (e.g., an adult (above age of 18) or late adolescent (between ages of 18-24)) or gender (e.g., male, female, or non-binary), among others. In at least partial concurrence with the time instance or the sessions during which the user is to perform tasks, the user may be on a medication to address the pathology. For example, the medication may include capsaicin, diflorasone, diphenhydramine, doxepin, hydrocortisone, prednisone, prednicarbate, pimecrolimus, tetracaine, tacrolimus, terbinafine, corticosteroids, vitamin D analogues, retinoids, calcineurin inhibitors, immunosuppressants, or antihistamines, among others. The medication may be of an effective amount (e.g., dosage or frequency), which may be sufficient to effect positive outcomes or reduction in symptoms (e.g., reduction in itch or propensity to scratch). The efficacy of the medication in terms of addressing the user's maladaptive behavior may be improved or enhanced from the concurrence with the tasks performed via the digital therapeutics application.

In some embodiments, there may be at least two groups for the trial to test the efficacy of the digital therapeutics application. The first group may include a set of users (e.g., including the user) for a treatment group. The users of the first group may be exposed to or provided with the tasks to be performed by the user via the digital therapeutics application (e.g., the application 125 or the Study App described herein). As discussed herein, the tasks may include, for example, go-no-go task, HRT task, and urge surfing task, or any combination thereof. The second group may include another set of users for a control group. The users of the second group may be exposed to or provided with a control therapy (sometimes herein referred to as a placebo or standard treatment).

The control therapy may exclude the tasks aimed at addressing the skin pathology and to be performed via the digital therapeutics application. In some embodiments, the control therapy may include a task performed via an application on a computing device that is inert with respect to the skin pathology or related symptoms. The task may include any task besides the go/no-go training, HRT, and urge surfing tasks as described herein for the digital therapeutic application. In some embodiments, the control therapy may include an inert version of go/no-go training in which users are presented with go stimuli, without any association with the skin pathology. The inert version of the go/no-go training may lack the no-go stimuli. The application for the control therapy may also lack HRT and the urge surfing tasks.

In some embodiments, the control therapy may include an inert version of HRT in which users are presented with prompts to perform tasks unrelated to the skin pathology. For instance, the inert version of the HRT may prompt users to perform breathing or running exercise, without any prompts on taking actions with respect to scratching or reflexive actions in response to itching. In some embodiments, the control therapy may include an inert version of urge surfing. For example, the inert version of urge surfing may lack any messaging to the user with respect to skin pathology. The control therapy may include other forms of cognitive behavioral therapy.

In some embodiments, the control therapy may include a care-as-usual (CAU) treatment regimen. The CAU treatment regimen may lack the digital therapeutics application described herein. For example, the CAU treatment regimen may have the user receive medication for the skin pathology on a scheduled basis (e.g., one to three times a day), and may include assessments of the skin pathology conducted in person or via a web application to evaluate symptoms. Baseline metrics may be obtained (e.g., via a computing device) from users of both the treatment group and the control group (e.g., receiving an inert version or CAU).

The method 1800 may include identifying or selecting a task from a set of tasks (1810). The task may be selected (e.g., by a computing device) from the set of tasks for a time instance or for a portion of a session at a given time instance. The set of tasks may be to lessen, alleviate, or otherwise reduce a frequency or intensity of the maladaptive behavior related to the skin pathology on the part of the user (e.g., scratching a skin affected by eczema) by having the user perform an action with the display of the computing device. The set of tasks from which the task is to be selected may include, for example, a go/no-go task to prompt the user to perform or withhold a first action, a habit reversal therapy task to prompt the user to perform the first action instead of performing a second action associated with the maladaptive behavior, and an urge surfing task to perform a third action for the duration of the maladaptive behavior, among others. The task may be selected using a number of factors, such as those detailed herein in Section A.

The method 1800 may include providing or presenting an indication to perform the selected task (1815). The digital therapeutics application running on the computing device can display, render, or otherwise present the indication to perform the tasking of one or both hands to hold the display in a specified manner. The indication may be presented via one or more user interface elements presented through the graphical user interface of the digital therapeutics application. The indication may direct the user to perform the task for a set period of time during the session. For example, the go-no-go task may be for 5 to 10 minutes, the HRT task may be for 2 to 3 minutes, and the urge surfing task may be for 2 to 5 minutes.

The method 1800 may include retrieving, identifying, otherwise obtaining a session metric (1820). The session metric may be associated with the user (e.g., the user 210) with respect to skin pathology or the related maladaptive behavior. The session metric may be obtained (e.g., by a computing device) subsequent to performing at least one of the time instances or sessions during which a task is to be performed by the user via the digital therapeutics application. The baseline measure may indicate the condition of the user with respect to the severity of the skin pathology and the associated maladaptive behavior on the user's daily life and activities, as a result of the performance of the task. The session metric may include, for example, a dermatology life quality index (DLQI) value, a peak pruritus numerical rating scale (PP-NRS) value, a patient-reported outcomes measurements information systems (PROMIS) value, a patient-oriented eczema measure (POEM) value, a psoriasis symptom inventory (PSI) value, a Scratch Intensity and Impact Scale (SIIS) value, a Sleep-Related Itch and Scratch Scale (value), Eczema Area and Severity Index (EASI), digital EASI (EASI-dig), Scoring in Atopic Dermatitis (SCORAD), Patient-Oriented SCORAD (PO-SCORAD), PASI (Psoriasis Area and Severity Index), or Self-Administered PASI (SA-PASI), a computerized cognitive assessment value, among others. The session metric may be of the same type of measurement as the baseline metric. The session metrics may be obtained from users of both the treatment group and the control group.

The method 1800 may include identifying or determining whether to continue (1825). The determination may be based on the set length (e.g., days, weeks, or years) of the trial, a set number of time instances during which to perform one or more tasks, or a set number of tasks to be provided to the user. For example, the set number of time instances may range between 2 weeks to 6 months, relative to the obtaining of the baseline metric. When the amount of time from the obtaining of the baseline metric exceeds the set length, the determination may be to stop providing additional tasks.

In contrast, when an amount of time from the obtaining of the baseline metric has not exceeded the set length, the determination may be to continue providing additional tasks and repeat from (1810). The selection or modification of task for the next time instance or session may be based on the performance of the task in the previous time instance or session. In some embodiments, the next task may be modified based on response data from the user while performing the task in the prior time instance or session. In some embodiments, the next task may be modified based on a score determined from the performance of the task in the prior time instance or session. The task may be modified, for example, by order of performance (e.g., with respect to other tasks), a time length to complete the task, and a difficulty of performing the task, among others. The selection or modification of task for the next time instance or session may be independent from the performance of the task in the previous time instance or session.

The method 1800 may include identifying or determining whether the session metric is an improvement over the baseline metric (1830). The improvement may correspond to the reduction in the frequency or the intensity of the maladaptive behavior related to the skin pathology. The improvement may be shown when the session metric is increased compared to the baseline metric by a first predetermined margin or when the session metric is decreased compared to the baseline metric by a second predetermined margin. The margin may identify or define a difference in value between the baseline and session metrics at which to determine that the user shows reduction in the frequency or the intensity of the maladaptive behavior related to the skin pathology. Whether the improvement is shown by increase or decrease may depend on the type of metric used to measure the user with respect to the skin pathology or the related maladaptive behavior. The margin may also depend on the type of metric used, and may in general correspond to the difference in value showing noticeable difference by the clinician or user with respect to the skin pathology or the maladaptive behavior, or showing a statistically significant result in the difference in the values between the baseline and session metrics.

In some embodiments, at least one of the first predetermined margin or the second predetermined margin may be calculated, generated, or otherwise determined (e.g., by a computing device). The first predetermined margin or the second predetermined margin may be based on the baseline and session metrics obtained from the treatment group or the control group of users. The margin may correspond to a difference between a change in baseline and session metrics in the treatment group versus a change in baseline and session metrics in the control group (e.g., receiving the control therapy) that is statistically significant. The difference may be based on any number of statistical tests, such as a t-test, a z-test, chi-squared test, among others.

The method 1800 may include determining that the reduction is shown when the session metric is determined to have improved over the baseline metric by the first predetermined margin (1835). In some embodiments, the reduction may occur when the session DLQI metric is increased compared to the baseline DLQI metric by the first predetermined threshold margin. In some embodiments, the reduction may occur when the session PP-NRS metric is decreased compared to the baseline PP-NRS metric by the first predetermined threshold margin. In some embodiments, the reduction may occur when the session PROMIS metric is increased compared to the baseline PROMIS metric by the first predetermined threshold margin. In some embodiments, the reduction may occur when the session POEM metric is decreased compared to the baseline POEM metric by the first predetermined threshold margin. In some embodiments, the reduction may occur when the session PSI metric is decreased compared to the baseline PSI metric by the first predetermined threshold margin.

In some embodiments, the reduction may occur when the session EASI metric (or EASI-dig) is decreased compared to the baseline EASI metric (or EASI-dig) by the first predetermined threshold margin. In some embodiments, the reduction may occur when the session SCORAD metric is decreased compared to the baseline SCORAD metric by the first predetermined threshold margin. In some embodiments, the reduction may occur when the session PO-SCORAD metric is decreased compared to the baseline PO-SCORAD metric by the first predetermined threshold margin. In some embodiments, the reduction may occur when the session SA-PASI metric is decreased compared to the baseline SA-PASI metric by the first predetermined threshold margin. In some embodiments, the reduction may occur when the session computerized cognitive assessment metric is decreased compared to the baseline computerized cognitive assessment metric by the first predetermined threshold margin.

The method 1800 may include determining that no reduction is shown when the session metric is determined to have not improved over the baseline metric by the second predetermined margin (1840). In some embodiments, no reduction may occur when the session DLQI metric is not increased compared to the baseline DLQI metric by the first predetermined threshold margin. In some embodiments, no reduction may occur when the session PP-NRS metric is not decreased compared to the baseline PP-NRS metric by the first predetermined threshold margin. In some embodiments, no reduction may occur when the session PROMIS metric is not increased compared to the baseline PROMIS metric by the first predetermined threshold margin. In some embodiments, no reduction may occur when the session POEM metric is not decreased compared to the baseline POEM metric by the first predetermined threshold margin. In some embodiments, no reduction may occur when the session PSI metric is not decreased compared to the baseline PSI metric by the first predetermined threshold margin.

In some embodiments, no reduction may occur when the session EASI metric (or EASI-dig) is not decreased compared to the baseline EASI metric (or EASI-dig) by the first predetermined threshold margin. In some embodiments, no reduction may occur when the session SCORAD metric is not decreased compared to the baseline SCORAD metric by the first predetermined threshold margin. In some embodiments, no reduction may occur when the session PO-SCORAD metric is not decreased compared to the baseline PO-SCORAD metric by the first predetermined threshold margin. In some embodiments, no reduction may occur when the session SA-PASI metric is not decreased compared to the baseline SA-PASI metric by the first predetermined threshold margin. In some embodiments, no reduction may occur when the session computerized cognitive assessment metric is not decreased compared to the baseline computerized cognitive assessment metric by the first predetermined threshold margin.

### Protocol - A Randomized, Controlled, Exploratory Basket Study to Evaluate Inhibitory Control, Habit Reversal Therapy and Urge Surfing to Disrupt Scratching in Atopic Dermatitis and Psoriasis

### 1. Protocol Summary

### Synopsis

CT-100 (e.g., the application 125) is a platform that provides interactive, software-based therapeutic components that may be used as part of a multimodal treatment in future software-based digital therapeutics. One class of CT-100 components are digital neuro-activation and modulation (DiNaMo^{™}) components. DiNaMo components target key neural systems (including, but not limited to, systems related to sensory, perceptual, affective, pain, attention, cognitive control and social and self-processing) to optimally improve a patient's health.

The inhibitory control, habit reversal therapy (HRT) and urge surfing combination DiNaMo aims to control urges (e.g., to scratch) through a combination of bottom-up and top-down approaches. Go/no-go (GNG) training (an inhibitory-control task) will help individuals implicitly train inhibitory-control skills to better resist oncoming urges. HRT disrupts these habitual activities by providing a physically incompatible alternate behavior to prevent participation in the undesired behavior. Finally, urge surfing helps train individuals to become comfortable experiencing an urge without giving in, by acknowledging this uncomfortable feeling and recognizing that it will eventually pass even if one does not participate in the desired behavior.

The purpose of the proposed study is to evaluate initial effects of the DiNaMo component (the Study Application [App]) on measures of undesired behavior intensity and related outcomes (inhibitory control and general quality of life [QoL]) in a variety of conditions, such as atopic dermatitis and psoriasis. Results derived from this research could be used as components within future prescription digital therapeutics (PDT).

### Objectives

To evaluate trends in comparison to a Digital Control arm for changes in undesired behavior intensity and related outcomes (inhibitory control and general QoL), as measured by indication specific measures, QoL measures and computerized cognitive assessments of inhibitory control. To explore the feasibility and acceptability of an at-home digital therapeutic using a combination of GNG, HRT and urge surfing training as measured by Study App engagement, the User Experience Questionnaire and qualitative interviews.

To evaluate the safety of the Study App. To explore the feasibility and acceptability of an at-home digital therapeutic using a combination of GNG, HRT and urge surfing training as measured by Study App engagement, the User Experience Questionnaire and qualitative interviews.

### Criteria for Evaluation

### Exploratory Endpoints

- Change from baseline to end of study in the Peak Pruritus Numerical Rating Scale (PP-NRS) or other measure of itching behavior
- Change from baseline to end of study in the Scratch Intensity and Impact Scale (SIIS) or other measure of scratching behavior
- Change from baseline to end of study in the Sleep-Related Itch and Scratch Scale (SRISS)
- Change from baseline to end of study in the Dermatology Life Quality Index (DLQI)
- Change in indication-specific measures from baseline to end of study
- Change in QoL symptoms assessed with the PROMIS^{®}-29+2 Profile v2.1 (PROMIS-29+2 Preference [PROPr]) scales from baseline to end of study
- Change in computerized cognitive assessment measure from baseline to end of study
- Engagement with the Study App as measured by metrics, such as daily App usage and daily time in the Study App
- Experience with the Study App as assessed by the User Experience Questionnaire and optional qualitative interviews after the Study App treatment period
- Proportion of participants with an improvement as measured by the Global Rating of Change (GRC) score at end of study

### Safety Endpoints

- Frequency and severity of adverse events (Aes), serious Aes and discontinuation from the study due to Aes
- Frequency and severity of Aes related to the worsening of symptoms related to the indication

### Study Design

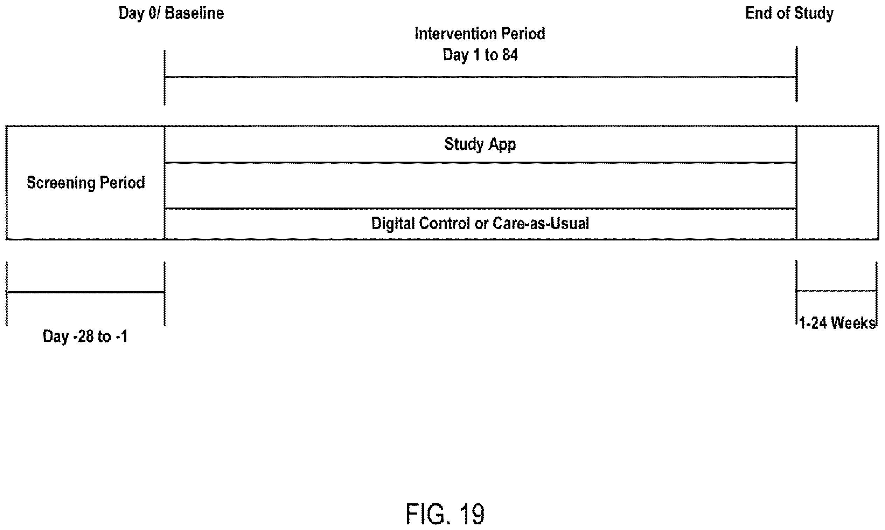

Referring now to FIG. 19, depicted is a timeline of the study design for evaluating inhibitory control, habit reversal therapy and urge surfing to disrupt scratching in atopic dermatitis and psoriasis.

Screening Period (Day -28 to Day -1): Participants will be screened for up to 14 days, including electronic informed consent. Assessments during this period will be performed (e.g., in person or remotely) according to the Schedule of Activities and Assessments (SoA). Screening and the Baseline Visit may occur on the same day.

Baseline Virtual Visit (Day 0): Eligible participants will be contacted for a Baseline Visit to review and confirm eligibility and perform identity verification. Participants will be considered eligible for study entry if they meet all inclusion and no exclusion criteria, based on investigator assessment. Eligible participants will be randomized during a virtual study visit on Day 0. Participants will be randomized 1:1 (e.g., Study App:Digital Control App or Study App:CAU) within their indication. Participants randomized to the Study-App treatment group will download and activate the App onto their personal primary iPhone or Android smartphone.

Intervention Period (Day 1 to Day 28-84): Study App group participants will be directed to access and perform tasks every day as directed by the Study App, for example, for 10-120 minutes per day, 3-10 times per day, 3-7 days per week, for 4-12 weeks. In some aspects, the participants will be directed to access and perform tasks every day over a period of 2-28 weeks, 2-20 weeks, 2-15 weeks, 2-12 weeks, 2-10 weeks, or 2-8 weeks. Control-group participants will receive a Digital Control App and perform tasks for approximately 1-10 minutes per day, 3-7 days per week for 4-12 weeks. Both groups will be assessed every 2-6 weeks during the 4-12-week Intervention Period. At the end of the treatment period, the Study App will be deactivated, Study-App-group participants will complete the User Experience Questionnaire.

Follow-up Period (up to 1-24 weeks): A subset of participants will be invited to complete an optional qualitative interview between end of treatment and 7 days later. Participants will not perform any activities within their respective app.

Planned Number of Participants: There will be at least 15-200 participants per indication, for a total of at least 30-400 participants..

### Study Entry Criteria

Inclusion Criteria: A participant will be eligible for entry into the study if all of the following criteria are met:
1. Fluent in written and spoken English, confirmed by ability to read, understand and sign the informed consent form (ICF).
2. Lives in the United States.
3. Aged 18 years or older.
4. Itch NRS ≥ 4 during baseline.
5. Meets indication-specific inclusion criteria, as reported by the study participant with adequate clinical documentation. (Documentation to be provided to the study team upon request.)
6. Has an active email address and is willing and able to receive and respond to email messages.
7. Has access to an internet connection during the study duration.
8. Willing and able to comply with the study protocol and assessments.
9. Is the sole user of an iPhone with an iPhone operating system (OS) 14 or later or a smartphone with an Android OS 10 or later for the duration of the study.
10. Is willing and able to receive Short Message Service (SMS) text messages and notifications on their smartphone.

Exclusion Criteria: A participant will not be eligible for study entry if any of the following criteria are met:
1. Pregnant or planning to become pregnant.
2. Visual, dexterity or cognitive deficit so severe that it precludes the use of an Appbased, reaction-time-based activity per investigator judgment.
3. Severe psychiatric disorder involving a history of psychosis (e.g., schizophrenia, bipolar disorder or severe personality disorders).
4. Psychiatric hospitalization in the past 6 months.
5. Participation in any research study (including studies on psychotherapy, mindfulness, cognitive training or pharmacological treatment) during the past 3 months.
6. Indication or change in primary-disease-specific medication within 30 days prior to entering the study.
7. Self-reported substance-use disorder within the past year.
8. Currently experiencing a skin infection.
9. Planning the introduction of new therapies (including studies on psychotherapy, mindfulness, cognitive training or pharmacological treatment) during the 7-39-week study period.
10. Anticipating a change in current pharmacological or psychotherapy treatment regimen during the 7-39-week study period.

Eligible Study App group participants will download and install the Study App onto their own smartphones at the Baseline Visit (Day 0).

Treatment Regimen: Study App: 4-12 weeks (10-120 minutes per day, 3-10 times per day, 3-7 days per week)

Study Duration: Participation in the study will last for approximately 7-39 weeks:
- Screening Period: Up to 2 weeks
- Intervention Period: 4-12 weeks
- Follow-up: Up to 1-24 weeks

### Sample Size

There will be at least 60-120 participants per indication, for a total of 120-240 participants. This sample size should be sufficient to measure the effect size with relative precision; however, this study is exploratory in nature and is used to estimate effect sizes.

### Statistical Analysis

Changes from baseline will be summarized using descriptive statistics. The PROMIS-29+2 subscale, the computerized cognitive assessment measure and DLQI, the PP-NRS or other itching measure, and the SIIS or other scratching measure, and SRISS change from baseline to end of study will be analyzed using an analysis-of-covariance (ANCOVA) model to estimate the difference between the 2 treatment arms. The model will include the change from baseline as the dependent variable and include treatment arm, indication and baseline values as covariates in the model. A 95% confidence interval (CI) and 2-sided p-value for the null hypothesis of no difference between the 2 arms will be provided. Least-squares means for the treatment effect will be provided. In addition, least-squares means, 95% Cis and p-values for the null hypothesis of no change from baseline will be provided for each treatment arm.

For indication specific measures such as the Patient-Oriented Eczema Measure (POEM), the Psoriasis Symptom Inventory (PSI), the Eczema Area and Severity Index (EASI), digital EASI (EASI-dig), Scoring in Atopic Dermatitis (SCORAD), Patient-Oriented SCORAD (PO-SCORAD), Psoriasis Area and Severity Index (PASI), or Self-Administered PASI (SAPASI, the change from baseline to end of study within each treatment arm and a 95% CI and 2-sided p-value for the null hypothesis of no difference between the treatment arms will be provided. The CI and the p-value will be based on t-distribution for 2 samples. A 95% CI for the change from baseline to end of study within each arm will be provided as well as a 2-sided p-value for the null hypothesis of no change from baseline using t-distribution for 1 sample (pre-post).

### Schedule of Activities and Assessments

Descriptive statistics will be used for the user experience as well as engagement and safety parameters.

| | **Screening/ Run-In** | **Follow-Up** | | | |
|---|---|---|---|---|---|
| **Study Day and Visit Window** | **Screening** | **Baseline** | **Every 2-6week** s | **EOS ET** | **Follow-up** |
| | **Day -14 to Day-1** | **Day 0 - 3 days** | **Every 14-28 days** | **Day 28-84 +3 days** | **Day 29-168 to Day 35-175** |
| **Study Visit Number** | NA | 1 | 3 | 5 | NA |
| **Remote Visit^{a}** | | X | | | |
| Informed consent | X | | | | |
| Medical History | X | | | | |
| Demographics | X | | | | |
| Inclusion/Exclusion Criteria | X | X | | | |
| ID Verification | | X | | | |
| Randomization | | X | | | |
| Study App Installation and Activation | | X | | | |
| Indication-specific Assessments^{b} | | X | X | X | X |
| PHQ-8 | | X | | | |
| Computerized performance assessment | | X | | X | X |
| PROPr | | X | X | X | X |
| GRC | | | | X | X |
| Study App Engagement | | X | X | X | |
| Concomitant Medications | X | X | X | X | |
| AE/SAE Review | X | X | X | X | X |
| User Experience Questionnaire | | | | X | |
| Study App Deactivation^{d} | | | | X | |
| Optional Qualitative Interview | | | | | X |
| PP-NRS | X | X | X | X | X |
| SIIS | X | X | X | X | X |
| SRISS | | X | X | X | X |
| DLQI | | X | X | X | X |

| | | | | | |
|---|---|---|---|---|---|
| Abbreviations: AE = adverse event; DLQI = Dermatology Life Quality Index; EOS = end of study; ET = early termination; GRC = Global Rating of Change; ID = identifier; NA = not applicable; PHQ-8 = Patient Health Questionnaire Depression Scale; PP-NRS = Peak Pruritus Numerical Rating Scale; PROMIS^{®}-29 = Patient-Reported Outcomes Measurement Information System - Profile v2.1; PROPr = PROMIS-29+2 Preference; SAE = serious adverse event; SIIS = Scratch Intensity and Impact Scale; SRISS = Slee-Related Itch and Scratch Scale ^{a} Remote visit(s) will be administered via video conference platform as necessary. ^{d} After completion of the intervention period, on Day 28-84, the respective App will become inert. After Day 28-84 or upon study withdrawal, participants will not have continued access to the content provided by the App. | | | | | |

### 2. Introduction

### Study Rationale

CT-100 is a platform that provides interactive, software-based therapeutic components that may be used as part of a multimodal treatment in future software-based digital therapeutics. One class of CT-100 components are digital neuro-activation and modulation (DiNaMo) components. DiNaMo components target key neural systems (including, but not limited to, systems related to sensory, perceptual, affective, pain, attention, cognitive control and social and self-processing) to optimally improve a patient's cognitive and mental health.

The inhibitory control, habit reversal therapy (HRT) and urge surfing combination DiNaMo aims to control urges (e.g., to scratch) through a combination of a bottom-up and topdown approaches. Go/no-go (GNG) training (an inhibitory control task) will help individuals implicitly train inhibitory-control skills to better resist oncoming urges. HRT disrupts these habitual activities by providing a physically incompatible alternate behavior to prevent participation in the undesired behavior. Finally, urge surfing helps train individuals to become comfortable experiencing an urge without giving in, by acknowledging this uncomfortable feeling and recognizing that it will eventually pass even if one does not participate in the desired behavior.

The purpose of the proposed study is to evaluate initial effects of the DiNaMo component (the Study Application [App]) on measures of undesired behavior intensity and related outcomes (inhibitory control and general quality of life [QoL]) in a variety of conditions, such as atopic dermatitis and psoriasis. Results derived from this research could be used as components within future digital therapeutics.

### Tasks

Inhibitory Control: Inhibitory control refers to the ability for an individual to withhold response based on impulse and habitual behavior. Inhibitory control can be assessed with a variety of cognitive tasks, but one of the most common is the GNG task. In this task, there are 2 possible stimuli: go, which prompts the individual to respond, and no-go, which prompts the individual to withhold from responding. The go stimulus appears more frequently, building up responding as the habitual action and thereby making the no-go condition more challenging.

Habit Reversal Therapy: HRT helps reduce unwanted repetitive behaviors. The first step is to become aware of cues that indicate the onset of a behavior one wishes to stop, and the second step is to replace this behavior with another physically incompatible behavior.

Urge Surfing: Urge surfing is a technique to manage undesirable habits, cravings or behaviors. One learns to withstand the discomfort that is presented by an urge that one does not act on and is taught to withstand it similar to a surfer riding a wave, which will pass on its own

### Application (CT-100-D-003 DiNaMo Component)

The CT-100-D-003 DiNaMo component (e.g., the application 125) will combine inhibitory-control training, using the GNG paradigm, with HRT and urge surfing. It is postulated that the GNG will prime the brain for plastic changes. These changes can be capitalized on by subsequent HRT lessons, to teach alternative behaviors in the face of itching, and urge surfing, to teach control over the anxiety that an unscratched itch can produce.

This training consists of regular, challenging exercises. In the current study, a treatment regimen of daily, 15-minute sessions split between GNG training, HRT and urge surfing will be conducted over a period of 4-12 weeks, which will have a similar total time on task and length of treatment compared to the previous studies in the literature.

The control group will use a Digital Control App. The Digital Control App is designed to be an inert version of GNG training. Participants will only be presented with neutral "Go" stimuli; there will be an absence of any disease-related "No-Go" stimuli, and these components of the task will not be mentioned. HRT and urge surfing will not be included in the control application.

Care-as-Usual Control: The care-as-usual (CAU) control group will only complete assessments and will not use the Study App. There may or may not be a CAU control group for the study.

### 3. Objectives and Endpoints

The study objectives are:
To evaluate trends in the Study App intervention group in comparison to the Digital Control App arm for changes in undesired behavior intensity and related outcomes (inhibitory control and general QoL), as measured by indication-specific measures, QoL measures and computerized cognitive assessments of inhibitory control.
To explore the feasibility and acceptability of an at-home digital therapeutic using a combination of GNG, HRT and urge surfing training as measured by Study App engagement, the User Experience Questionnaire and qualitative interviews.

This study is exploratory in nature and does not seek to evaluate specific statistical endpoints. Study endpoints are listed in Table 1.

**Table 1: Study Endpoints**

| | Exploratory Endpoints |
|---|---|
| | • Change from baseline to end of study in the Peak Pruritus Numerical Rating Scale (PP-NRS) or other measure of itching behavior |
| | • Change from baseline to end of study in the Scratch Intensity and Impact Scale (SIIS) or other measure of scratching behavior |
| | • Change from baseline to end of study in the Sleep-Related Itch and Scratch Scale (SRISS) |
| | • Change in indication-specific measures from baseline to end of study |
| | • Change in quality-of-life symptoms assessed with the PROMIS^{®}-29+2 Profile v2.1 (PROMIS-29+2 Preference [PROPr]) scales (including mood, fatigue, pain interference, sleep, ability to participate in social functioning and cognitive function) from baseline to end of study |
| | • Change in computerized cognitive assessment measures from baseline to end of study for each group (DiNaMo and control) |
| | • Engagement with the Study App as measured by metrics, such as daily App usage and daily time in the Study App |
| | • Experience with the Study App as assessed by the User Experience Questionnaire and optional qualitative interviews after the Study App treatment period |
| | • Proportion of participants with an improvement as measured by the • Global Rating of Change (GRC) score at end of study. |

| | Safety Endpoints |
|---|---|
| • | Frequency and severity of adverse events (Aes), serious Aes and discontinuation from the study due to Aes |
| • | Frequency and severity of Aes related to the worsening of symptoms related to the indication. |

### 4. Study Design

### Overall Design

With reference to FIG. 19, this is a randomized, virtual study to evaluate the Study App compared to a Digital Control App (or CAU) in participants with atopic dermatitis and psoriasis. The study consists of an up-to-2-week screening period, a -12-week intervention period and 1-24-week follow-up. Participants that meet eligibility criteria will be randomized in the study on Day 0 (Baseline Visit).

Screening Period (Day -14 to Day 0): Participants will be screened for up to 14 days, including electronic informed consent. Assessments during this period will be performed remotely according to the Schedule of Activities and Assessments (SoA). Participants will be considered eligible if they meet all inclusion criteria and no exclusion criteria, based on investigator assessment. Screening and the Baseline Visit may occur on the same day.

Baseline Virtual Visit (Day 0): Eligible participants will be contacted for a Baseline Visit to review and confirm eligibility and perform identity verification. Participants will be considered eligible for study entry if they meet all inclusion and no exclusion criteria, based on investigator assessment.

Eligible participants will be randomized during a virtual study visit on Day 0. Participants will be randomized 1: 1 (Study App:Control) within their indication. Participants randomized to the Study App treatment group will download and activate the App onto their personal primary iPhone or Android smartphone.

Intervention Period (Day 1 to Day 28): Study App group participants will be directed to access and perform tasks every day as directed by the Study App for up to 10-60 minutes per day, 3-7 days a week for 4-12 weeks. Assessments will occur according to the Schedule of Activities and Assessments (SoA). Control group participants will receive a Digital Control App (or CAU). Both groups will be assessed every 2-6 weeks during the -12-week Intervention Period.

At the end of the treatment period, the Study App will be deactivated, and Study App group participants will complete the User Experience Questionnaire.

Follow-up Period (up to 1-24 weeks): Participants will complete follow-up assessments according to the SoA. A subset of participants will be invited to complete an optional qualitative interview. Participants will not perform any activities within the Study App (see Section 6.2).

### Rationale for Study Design

This study is designed to evaluate the initial effects of the Study App on undesired behavior intensity and related outcomes. Participants will be assessed based on validated, standard, participant-rated outcomes. Participant engagement with the Study App will be evaluated based on participant-usage data captured within the Study App. Participants will also be evaluated for safety throughout the duration of the study. The scales and assessments are described below.

### End of Study Definition

The end of the study is defined as the date of the last contact, or the date of final contact attempt, for the last participant completing or withdrawing from the study. For the purposes of this study, participants who complete the assessments at Day 28-84 (+ 3) (End of Study) will be defined as study completers.

### 5. Study Population

Participants will be assigned a unique participant identifier (ID) at screening upon signing the informed consent form (ICF). Prospective approval of protocol deviations to recruitment and enrollment criteria, also known as protocol waivers or exemptions, is not permitted.

### Inclusion Criteria

A participant will be eligible for study entry if all of the following criteria are met:
1. Fluent in written and spoken English, confirmed by ability to read, understand and sign the ICF.
2. Lives in the United States.
3. Aged 18 years or older.
4. Itch Numerical Rating Scale (NRS) ≥ 4 during baseline.
5. Meets indication-specific inclusion criteria, as reported by the study participant with adequate clinical documentation. (Documentation will be provided to the study team upon request.)
6. Has an active email address and is willing and able to receive and respond to email messages.
7. Has access to an internet connection during the study duration.
8. Willing and able to comply with the study protocol and assessments.
9. Is the sole user of an iPhone with an iPhone operating system (OS) 14 or later or a smartphone with an Android OS 10 or later for the duration of the study.
10. Is willing and able to receive Short Message Service (SMS) text messages and notifications on their smartphone.

### Exclusion Criteria

A participant will not be eligible for study entry if any of the following criteria are met:
1. Pregnant or planning to become pregnant.
2. Visual, dexterity or cognitive deficit so severe that it precludes the use of an appbased, reaction-time-based activity per investigator judgment.
3. Severe psychiatric disorder involving a history of psychosis (e.g., schizophrenia, bipolar disorder or severe personality disorders.)
4. Psychiatric hospitalization in the past 6 months.
5. Participation in any research study (including studies on psychotherapy, mindfulness, cognitive training or pharmacological treatment) during the past 3 months.
6. Indication or change in primary-disease-specific medication within 30 days prior to entering the study.
7. Self-reported substance use disorder within the past 1 year.
8. Currently experiencing a skin infection.
9. Planning the introduction of new therapies (including studies on psychotherapy, mindfulness, cognitive training or pharmacological treatment) during the 7-39-week study period.
10. Anticipating a change in current pharmacological or psychotherapy treatment regimen during the 7-39-week study period.

Lifestyle Considerations: Participants should have routine access to their smartphones for the duration of the trial.

### 6. Study Interventions

Study App: The study intervention under evaluation is the CT-100 DiNaMo component, a digital mobile application (e.g., the application 125). Participants randomized to this group will download and install the Study App onto their own smartphones at the Baseline Visit (Day 0) and use the Study App daily for DiNaMo training over the -12-week intervention period.

Digital Control: Participants randomized to the Digital Control group will download and install the Digital Control App to their own smartphone at the Baseline Visit (Day 0) and use the Digital Control App daily over the 4-12-week intervention period. Certain participants may be randomized to the CAU control group and will complete study assessments only over the 4-week intervention period.

App Download and Activation: During the Baseline Visit, study personnel will assist the participants randomized to download, install and activate their respective App. Instructions for installation and activation can be found in the CT-100-D-003 Study App Site Instructions. Only participants who are enrolled in the study may activate the App.

App De-Activation and Un-Installation: After completion of the intervention period (Day X), the Study App will become inert and become unusable for participants. Site personnel will inform participants who complete the study or terminate early to uninstall the Study App.

Measures to Minimize Bias: Randomization: Participants within each indication under study will be randomly assigned in a 1:1 ratio to the Study App or Digital Control App. To mitigate participant expectation, participants in this trial will be blinded to the efficacy hypothesis. This means that eligible participants will be informed by trial site staff that: a) they will be randomized to one of two digital therapeutic treatments during the trial, and b) the purpose of the trial is to compare the effectiveness of these two digital therapeutic treatments, which may or may not improve indication-related symptoms. No references to the "Digital Control App" or "Control App" should be made to the participant; both interventions should only be referred to as the Study App.

Study Intervention Adherence: Participants will be told to use their respective App (Study App or Digital Control App) as instructed by the App. Compliance with this regimen will not be defined for this study. However, the level of App engagement will be measured.

Protocol Deviations: Note that an out-of-window visit is not considered a protocol deviation unless the site does not demonstrate effort to remind the participant of the next appointment or does not take corrective action to change the participant's behavior.

Concomitant Therapy: Participants will continue to use their prescribed therapies while enrolled in this study. Participants will self-report any changes to concomitant therapies through the end of the follow-up period.

### 7. Study Assessments and Procedures

Study assessments and procedures, including their timing, are summarized in the SoA. Adherence to the study design requirements, including those specified in the SoA, is essential and required for study conduct. Protocol waivers or exemptions are not allowed. Every effort should be made to ensure that the protocol-required assessments and procedures are completed as described. Study and safety assessments are described below.

Study Assessments: The following assessment scales are used in this study at the times as provided in the SoA

Screening Survey: The Screening Survey is a non-validated survey describing the DiNaMo daily exercises, asking the participant to reflect on whether they are motivated and willing to commit to approximately 10-60 minutes daily of app-delivered tracking and/or exercises for 4-12weeks. The survey also includes questions on demographics, medical history, medications, eligibility criteria and pregnancy status. This questionnaire is completed by the participant.

Patient Health Questionnaire Depression Scale: The Patient Health Questionnaire Depression Scale (PHQ-8) is an 8-item self-report measure to establish mood-disorder diagnoses as well as grade mood symptom severity. This scale is completed electronically by the participant.

Computerized Performance Measures: The computerized cognitive-performance assessment(s) will measure inhibitory control. This/These cognitive assessment(s) will be conducted during the Baseline Visit and during the ET/EOS Visit.

Itch Numerical Rating Scale: The Peak Pruritus (Itch) NRS (PP-NRS) is a single-item, patient-reported outcome of itch severity. Participants rate the "worst" itch in the previous 24 hours on a scale of 0 to 10, with 10 being the "worst itch imaginable". Itch may also be measured through a diary tracking mechanism or itching cognitions questionnaire.

Scratch Intensity and Impact Scale: The Scratch Intensity and Impact Scale (SIIS) is a 13-item questionnaire using 5-point Likert type measures ranging from 0 (never) to 4 (always). Scratching can also be measured by wearable sensors that detect scratching, a diary tracking mechanism, or scratch pleasure scales.

Sleep-Related Itch and Scratch Scale: the Sleep-Related Itch and Scratch Scale (SRISS) is a 16-item questionnaire using a 5-point Liekert-type scale ranging from 0 (never) to 4 (always) to assess impact of itching and scratching on various aspects of sleep over the last 7 days.

Dermatology Life Quality Index: The Dermatology Life Quality Index (DLQI) is a simple, practical questionnaire that evaluates the impact of skin disease and its treatment on the QoL of participants.

PROMIS^{®}-29+2 Profile v2.1: PROMIS-29 is part of the Patient-Reported Outcomes Measurement Information System (PROMIS). PROMIS-29 is a short-form assessment that contains 4 items from each of the 7 PROMIS domains (Mood, Physical Function, Pain Interference, Depressive Symptoms, Fatigue, Sleep Disturbance and Ability to Participate in Social Roles and Activities) plus 1 pain intensity question (0 to 10 NRS) and 2 cognitive-functioning questions. The PROMIS-29 is universal rather than disease specific (i.e., can assess health from patients regardless of disease or condition) and is intended for adults (ages greater than 18 years). Scores are produced for all 7 domains. The domains are assessed over the past 7 days. The PROMIS-29 has been widely administered and validated in a range of populations and settings. This electronic questionnaire is completed by the participant. It takes approximately 7 minutes to complete. The PROMIS 29+2 also permits the calculation of the PROMIS Preference (PROPr) score. This allows for the calculation of a single QoL summary score using the PROMIS subscales. The calculation lies on a scale from 0 (dead) to 1 (full health).

User Experience Questionnaire (and Optional Qualitative Interview): The User Experience Questionnaire is a questionnaire developed to understand a participant's experience with the Study App during the intervention phase. The questionnaire will ask questions related to the perceived enjoyment, challenges and related user experience and will not contain questions related to clinical outcomes. This questionnaire will be completed electronically by the participant. It takes approximately 7 minutes to complete.

Additionally, a subset of participants may participate in phone- or video-conference-based qualitative user interviews. These interviews will gather additional information about the user's experience with the App, such as favorite App features, usability of the features, challenges related to the intervention or any other feedback from regularly interacting with the App.

Global Rating of Change: The Global Rating of Change (GRC) is a self-reported, single-item, 10-point Likert scale used to assess the participant's rating of overall improvement with their indication after study intervention. This item will be completed electronically by the participant.

Patient-Oriented Eczema Measure (POEM): The POEM is a 7-item, selfassessment measurement tool that captures the fluctuating and chronic nature of atopic eczema and provides a comprehensive assessment of patient symptoms.

Psoriasis Symptom Inventory (PSI): The PSI is an 8-item, patient-reported outcome measure for assessing the severity of plaque psoriasis symptoms. Each item is scored on a 5-point scale.

The Eczema Area and Severity Index (EASI): The EASI is a physician-assessed tool that estimates atopic dermatitis disease severity. Four regions (head/neck, upper limbs, trunk, and lower limbs) are assessed for disease severity by assessing the degree of severity of each of four symptoms (erythema, induration, excoriations, and lichenification) on a scale from 0 to 3 (none to severe). This value is then multiplied by approximate area affected score, ranging from 1 (<10%) to 6 (>90%), and then multiplied by a scale factor to account for the proportionate size of each region. All four are summed together to provide a comprehensive score of disease severity. EASIdig uses digital images to automatically and digitally assess and approximate the EASI score without requiring physician overview.

The SCORing Atopic Dermatitis assessment (SCORAD) is a physician-assessed tool that estimates atopic dermatitis disease severity. The sites affected by the disease are shaded on a body to give an approximation of area affected. For each area intensity in that area is assessed on a scale of 0 (none) to 3 (severe). Severity signs are a comprehensive assessment that takes into consideration redness, swelling, oozing, scratch marks, lichenification, and dryness. Finally, subjective symptoms are assessed, calculating itch and sleepiness on a 10-point scale ranging from 0 (no itch/sleepiness) to 10 (worst imaginable). The final score is area affected/5 + 7*(summation of all severity scores)/2 + (sum of subjective symptoms score). The Patient-Oriented SCORAD (PO-SCORAD) is a patient-reported version of the same tool.

The Psoriasis Area and Severity Index (PASI) is a physician-assessed tool that estimates psoriasis disease severity. Four regions (head/neck, upper limbs, trunk, and lower limbs) are assessed for disease severity by assessing the degree of severity of each of three symptoms (redness, thickness, and scaling) on a scale from 0 to 4 (absent to very severe). These severity scores are summed together for each region and then then multiplied by a scale factor to account for the proportionate size of each region. This value is then multiplied by approximate area affected score, ranging from 0 (none) to 6 (90-100%). Finally, these four area scores are added together to obtain the final PASI score. The Self-Administered PASI (SAPASI) or Patient-Oriented PASI (PO-PASI) is a patient-reported version of the same tool.

### 8. Statistical Considerations

The statistical analysis plan (SAP) will be finalized prior to database lock and will include a more technical and detailed description of the statistical analyses described in this section.

### Sample Size Determination

Approximately 60-120 participants will be randomized to each indication under study in a 1:1 (Study App:Digital Control App or CAU) ratio, for a total of 120-240 participants. This sample size should be sufficient to measure the effect size with relative precision; however, this study is exploratory in nature and is used to estimate effect sizes.

### Analyses Sets

For the purposes of analysis, the following analysis sets are defined:

| **Analysis Set** | **Description** |
|---|---|
| Enrolled | All participants who signed the informed consent form |
| | This analysis set will be used for disposition. |
| Intent-to-Treat (ITT) | All randomized participants, based on the assigned arm in the randomization and recorded in the database, regardless of successful activation or use of the Study App. Participants treated without being randomized will not be considered for the ITT Set. |
| | This analysis set will be used as a supportive analysis for some of the efficacy endpoints. |
| Modified Intent-to-Treat (mITT) | All ITT participants, based on the assigned arm in the randomization and recorded in the database, who have evaluable baseline measurements. |
| | Participants in the Study App will be included in the mITT if the Study App was activated and they have used the application at least once. |
| | This will be the main analysis set for all efficacy analyses and will be used to summarize baseline and demography. |
| Per-Protocol (PP) | All randomized participants who completed the 4-12-week treatment and who did not have a major protocol deviation. This analysis set will be used as a supportive analysis for the selected efficacy endpoint. |
| | Participants with the following deviations will be excluded from the PP set: |
| | 1. Participants who received intervention different from the randomized intervention |
| | 2. Participants who do not meet the inclusion/exclusion criteria |
| | 3. Participants who used disallowed medication |
| | 4. Participants who meet discontinuation criteria but have not discontinued |
| | Additional criteria for exclusion from the PP set may be defined in the statistical analysis plan. |
| Safety | All participants randomized. Participants will be analyzed according to the intervention they received. This analysis set will be used for all safety analyses. |

### General Considerations

Descriptive statistics will include n, means and standard deviations, medians, and minimums and maximums for continuous variables. For categorical variables, Ns and percentages will be reported. Unless otherwise specified, tabulations will be done by treatment arm.

95% confidence intervals (Cis) and statistical tests will be performed for some of the efficacy endpoints and will be detailed as part of the description of planned analysis for each of the endpoints.

Data capture methods will be programmed to minimize missing data (e.g., an answer must be given to advance to the next assessment question). The SAP will address handling of incomplete questionnaires in which there are domains or total. In cases that a full questionnaire or a visit are missing, no imputations will be done, and only observed data will be included in the analysis.

### Participant's Disposition

This analysis will be done on the Enrolled Analysis Set. The number of participants who were enrolled, screen failed, randomized and completed the study will be presented. The number of subjects in each analysis set will be presented by treatment arm within indication.

### Demographics and Baseline Characteristics

In order to assess the comparability of the 2 arms at baseline, demographic and baseline characteristic data will be summarized by treatment group within each indication and overall by treatment. These summaries will be presented for the Modified Intent-to-Treat (mITT) Set. If there is a difference of greater than 5 participants in the total number of participants between the mITT and the Safety, Intent-to-Treat and Per-Protocol Analysis Sets, the summaries will be presented for these sets.

### Exploratory Endpoint Analysis

The SAP will elaborate on derivation of endpoints, in particular for the questionnaires with several domains. The analysis of those endpoints will be performed by treatment arm using the mITT Set. The analysis will be repeated for the Per-Protocol Set. Absolute value, changes from baseline to intermediate weeks (when applicable) and changes to end of study will be summarized using descriptive statistics.

The PROMIS-29+2 subscale and PROPr, the computerized cognitive assessment measure(s), and DLQI and PP-NRS change from baseline to end of study will be analyzed using an analysis-of-covariance model to estimate the difference between the 2 treatment arms. The model will include the change from baseline as the dependent variable, and include treatment arm, indication and baseline value as covariates to the model. A 95% CI and 2-sided p-value for the null hypothesis of no difference between the 2 arms will be provided. Least-squares means for the treatment effect will be provided. In addition, least-squares means, 95% Cis and p-value for the null hypothesis of no change from baseline will be provided for each treatment arm.

For the Patient-Oriented Eczema Measure (POEM) and the Psoriasis Symptom Inventory (PSI) or other indication-specific measures, the change from baseline to end of study within each treatment arm and 95% CI and 2-sided p-value for the null hypothesis of no difference between the treatment arms will be provided. The CI and the p-value will be based on t-distribution for 2 samples. A 95% CI for the change from baseline to end of study within each arm will be provided as well as a 2-sided p-value for the null hypothesis of no change from baseline using t-distribution for 1 sample (pre-post).

For measures that were evaluated at baseline, every 2-6 weeks and end of study, graphs of mean (standard error) over time for change from baseline by treatment arm and within indication will be generated using the mITT Set. Descriptive statistics will be used for the user experience as well as engagement and safety parameters.

### Example 1: Use of CT-100-D-003 DiNaMo in a Digital Therapeutics to Target Maladaptive Behaviors Associated with Skin Pathologies in General

In one example, the CT-100-D-003 DiNaMo application (also referred herein as the application) (e.g., the application 125) will be given to individuals with skin pathologies. The individuals with skin pathologies will be selected based on having an Itch Numerical Rating Scale (NRS) of ≥ 4. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, or PROMIS, among others. The improvement in at least one indication-related symptom in individuals receiving the application is expected to be statistically significant relative to any improvement observed in the corresponding indication-related symptom in individuals receiving the Digital Control or CAU.

### Example 2: Use of CT-100-D-003 DiNaMo in a Digital Therapeutics to Target Maladaptive Behaviors Associated with Atopic Dermatitis

In one example, the CT-100-D-003 DiNaMo application (also referred herein as the application) (e.g., the application 125) will be given to individuals with skin pathologies. The individuals with skin pathologies will be selected based on having an Itch Numerical Rating Scale (NRS) of ≥ 4 and in particular diagnosed with or at risk of atopic dermatitis. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, PROMIS, POEM, SCORAD, PO-SCORAD, among others. The improvement in the at least one indication-related symptom in individuals receiving the application is expected to be statistically significant relative to any improvement observed in the corresponding indication-related symptom in individuals receiving the Digital Control or CAU.

### Example 3: Use of CT-100-D-003 DiNaMo in a Digital Therapeutics to Target Maladaptive Behaviors Associated with Psoriasis

In one example, the CT-100-D-003 DiNaMo application (also referred herein as the application) (e.g., the application 125) will be given to individuals with skin pathologies. The individuals with skin pathologies will be selected based on having an Itch Numerical Rating Scale (NRS) of ≥ 4 and in particular diagnosed with or at risk of psoriasis. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, PROMIS, PSI, or PASI, among others. The improvement in the at least one indication-related symptom in individuals receiving the application is expected to be statistically significant relative to any improvement observed in the corresponding indication-related symptom in individuals receiving the Digital Control or CAU

### Example 4: Use of CT-100-D-003 DiNaMo in a Digital Therapeutics to Target Maladaptive Behaviors Associated with Contact Dermatitis

In one example, the CT-100-D-003 DiNaMo application (also referred herein as the application) (e.g., the application 125) will be given to individuals with skin pathologies. The individuals with skin pathologies will be selected based on having an Itch Numerical Rating Scale (NRS) of ≥ 4, and in particular diagnosed with or at risk of contact dermatitis. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, or PROMIS, among others. The improvement in the at least one indication-related symptom in individuals receiving the application is expected to be statistically significant relative to any improvement observed in the corresponding indication-related symptom in individuals receiving the Digital Control or CAU.

### Example 5: Use of CT-100-D-003 DiNaMo in a Digital Therapeutics to Target Maladaptive Behaviors Associated with Urticaria

In one example, the CT-100-D-003 DiNaMo application (also referred herein as the application) (e.g., the application 125) will be given to individuals with skin pathologies. The individuals with skin pathologies will be selected based on having an Itch Numerical Rating Scale (NRS) of ≥ 4 and in particular diagnosed with or at risk of urticaria. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, or PROMIS, among others. The improvement in the at least one indication-related symptom in individuals receiving the application is expected to be statistically significant relative to any improvement observed in the corresponding indication-related symptom in individuals receiving the Digital Control or CAU.

### Example 6: Use of CT-100-D-003 DiNaMo in a Digital Therapeutics to Target Maladaptive Behaviors Associated with Prurigo Nodularis

In one example, the CT-100-D-003 DiNaMo application (also referred herein as the application) (e.g., the application 125) will be given to individuals with skin pathologies. The individuals with skin pathologies will be selected based on having an Itch Numerical Rating Scale (NRS) of ≥ 4 and in particular diagnosed with or at risk of prurigo nodularis. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, or PROMIS, among others. The improvement in the at least one indication-related symptom in individuals receiving the application is expected to be statistically significant relative to any improvement observed in the corresponding indication-related symptom in individuals receiving the Digital Control or CAU.

### Example 7: Use of CT-100-D-003 DiNaMo in a Digital Therapeutics to Target Maladaptive Behaviors Associated with Lichen Planus

In one example, the CT-100-D-003 DiNaMo application (also referred herein as the application) (e.g., the application 125) will be given to individuals with skin pathologies. The individuals with skin pathologies will be selected based on having an Itch Numerical Rating Scale (NRS) of ≥ 4 and in particular diagnosed with or at risk of lichen planus. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, or PROMIS, among others. The improvement in the at least one indication-related symptom in individuals receiving the application is expected to be statistically significant relative to any improvement observed in the corresponding indication-related symptom in individuals receiving the Digital Control or CAU.

### Example 8: Use of CT-100-D-003 DiNaMo in a Digital Therapeutics to Target Maladaptive Behaviors Associated with Acne Vulgaris

In one example, the CT-100-D-003 DiNaMo application (also referred herein as the application) (e.g., the application 125) will be given to individuals with skin pathologies. The individuals with skin pathologies will be selected based on having an Itch Numerical Rating Scale (NRS) of ≥ 4 and in particular diagnosed with or at risk of acne vulgaris. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, or PROMIS, among others. The improvement in the at least one indication-related symptom in individuals receiving the application is expected to be statistically significant relative to any improvement observed in the corresponding indication-related symptom in individuals receiving the Digital Control or CAU.

### Example 9: Use of CT-100-D-003 DiNaMo in a Digital Therapeutics to Target Maladaptive Behaviors Associated with Drug-Induced Rashes

In one example, the CT-100-D-003 DiNaMo application (also referred herein as the application) (e.g., the application 125) will be given to individuals with skin pathologies. The individuals with skin pathologies will be selected based on having an Itch Numerical Rating Scale (NRS) of ≥ 4 and in particular diagnosed with or at risk of drug-induced rashes. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, or PROMIS, among others. The improvement in the at least one indication-related symptom in individuals receiving the application is expected to be statistically significant relative to any improvement observed in the corresponding indication-related symptom in individuals receiving the Digital Control or CAU

### Example 10: Use of CT-100-D-003 DiNaMo in a Digital Therapeutics to Target Maladaptive Behaviors Associated with Xerosis

In one example, the CT-100-D-003 DiNaMo application (also referred herein as the application) (e.g., the application 125) will be given to individuals with skin pathologies. The individuals with skin pathologies will be selected based on having an Itch Numerical Rating Scale (NRS) of ≥ 4 and in particular diagnosed with or at risk of xerosis. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, or PROMIS, among others. The improvement in the at least one indication-related symptom in individuals receiving the application is expected to be statistically significant relative to any improvement observed in the corresponding indication-related symptom in individuals receiving the Digital Control or CAU.

### Example 11: Use of CT-100-D-003 DiNaMo in a Digital Therapeutics to Target Maladaptive Behaviors Associated with Uremia

In one example, the CT-100-D-003 DiNaMo application (also referred herein as the application) (e.g., the application 125) will be given to individuals with skin pathologies. Individuals with skin pathologies will be selected based on having an Itch Numerical Rating Scale (NRS) of ≥ 4 and in particular diagnosed with or at risk of uremia. The treatment will be 2-28 weeks long in accordance with the Protocol. It is expected that individuals receiving the treatment will show an improvement in at least one indication-related symptom, as measured by Computerized Performance Measures, PP-NRS, SIIS, SRISS, DLQI, or PROMIS, among others. The improvement in the at least one indication-related symptom in individuals receiving the application is expected to be statistically significant relative to any improvement observed in the corresponding indication-related symptom in individuals receiving the Digital Control or CAU.

### C. Network and Computing Environment

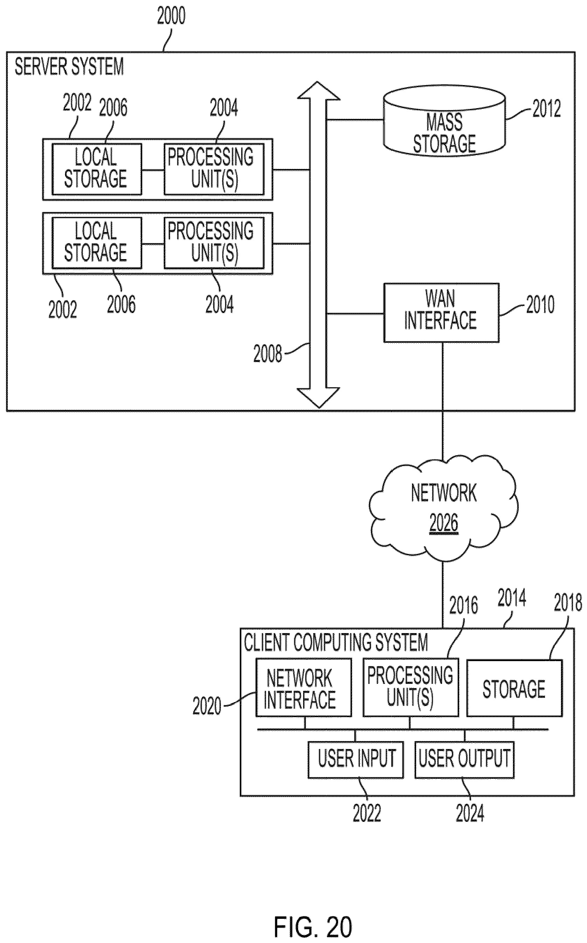

Various operations described herein can be implemented on computer systems. FIG. 20 shows a simplified block diagram of a representative server system 2000, client computer system 2014, and network 2026 usable to implement certain embodiments of the present disclosure. In various embodiments, server system 2000 or similar systems can implement services or servers described herein or portions thereof. Client computer system 2014 or similar systems can implement clients described herein. The system 100 described herein can be similar to the server system 2000. Server system 2000 can have a modular design that incorporates a number of modules 2002 (e.g., blades in a blade server embodiment); while two modules 2002 are shown, any number can be provided. Each module 2002 can include processing unit(s) 2004 and local storage 2006.

Processing unit(s) 2004 can include a single processor, which can have one or more cores, or multiple processors. In some embodiments, processing unit(s) 2004 can include a general-purpose primary processor as well as one or more special-purpose co-processors such as graphics processors, digital signal processors, or the like. In some embodiments, some or all processing units 2004 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 2004 can execute instructions stored in local storage 2006. Any type of processors in any combination can be included in processing unit(s) 2004.

Local storage 2006 can include volatile storage media (e.g., DRAM, SRAM, SDRAM, or the like) and/or non-volatile storage media (e.g., magnetic or optical disk, flash memory, or the like). Storage media incorporated in local storage 2006 can be fixed, removable, or upgradeable as desired. Local storage 2006 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 2004 need at runtime. The ROM can store static data and instructions that are needed by processing unit(s) 2004. The permanent storage device can be a non-volatile read-and-write memory device that can store instructions and data even when module 2002 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 2006 can store one or more software programs to be executed by processing unit(s) 2004, such as an operating system and/or programs implementing various server functions such as functions of the system 100 or any other system described herein, or any other server(s) associated with system 100 or any other system described herein.

"Software" refers generally to sequences of instructions that, when executed by processing unit(s) 2004, cause server system 2000 (or portions thereof) to perform various operations, thus defining one or more specific machine embodiments that execute and perform the operations of the software programs. The instructions can be stored as firmware residing in read-only memory and/or program code stored in non-volatile storage media that can be read into volatile working memory for execution by processing unit(s) 2004. Software can be implemented as a single program or a collection of separate programs or program modules that interact as desired. From local storage 2006 (or non-local storage described below), processing unit(s) 2004 can retrieve program instructions to execute and data to process in order to execute various operations described above.

In some server systems 2000, multiple modules 2002 can be interconnected via a bus or other interconnect 2008, forming a local area network that supports communication between modules 2002 and other components of server system 2000. Interconnect 2008 can be implemented using various technologies, including server racks, hubs, routers, etc.

A wide area network (WAN) interface 2010 can provide data communication capability between the local area network (e.g., through the interconnect 2008) and the network 2026, such as the Internet. Other technologies can be used to communicatively couple the server system with the network 2026, including wired (e.g., Ethernet, IEEE 802.3 standards) and/or wireless technologies (e.g., Wi-Fi, IEEE 802.11 standards).

In some embodiments, local storage 2006 is intended to provide working memory for processing unit(s) 2004, providing fast access to programs and/or data to be processed while reducing traffic on interconnect 2008. Storage for larger quantities of data can be provided on the local area network by one or more mass storage subsystems 2012 that can be connected to interconnect 2008. Mass storage subsystem 2012 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 2012. In some embodiments, additional data storage resources may be accessible via WAN interface 2010 (potentially with increased latency).

Server system 2000 can operate in response to requests received via WAN interface 2010. For example, one of modules 2002 can implement a supervisory function and assign discrete tasks to other modules 2002 in response to received requests. Work allocation techniques can be used. As requests are processed, results can be returned to the requester via WAN interface 2010. Such operation can generally be automated. Further, in some embodiments, WAN interface 2010 can connect multiple server systems 2000 to each other, providing scalable systems capable of managing high volumes of activity. Other techniques for managing server systems and server farms (collections of server systems that cooperate) can be used, including dynamic resource allocation and reallocation.

Server system 2000 can interact with various user-owned or user-operated devices via a wide-area network such as the Internet. An example of a user-operated device is shown in FIG. 20 as client computing system 2014. Client computing system 2014 can be implemented, for example, as a consumer device such as a smartphone, other mobile phone, tablet computer, wearable computing device (e.g., smart watch, eyeglasses), desktop computer, laptop computer, and so on.

For example, client computing system 2014 can communicate via WAN interface 2010. Client computing system 2014 can include computer components such as processing unit(s) 2016, storage device 2018, network interface 2020, user input device 2022, and user output device 2024. Client computing system 2014 can be a computing device implemented in a variety of form factors, such as a desktop computer, laptop computer, tablet computer, smartphone, other mobile computing device, wearable computing device, or the like.

Processing unit 2016 and storage device 2018 can be similar to processing unit(s) 2004 and local storage 2006 described above. Suitable devices can be selected based on the demands to be placed on client computing system 2014. For example, client computing system 2014 can be implemented as a "thin" client with limited processing capability or as a highpowered computing device. Client computing system 2014 can be provisioned with program code executable by processing unit(s) 2016 to enable various interactions with server system 2000.

Network interface 2020 can provide a connection to the network 2026, such as a wide area network (e.g., the Internet) to which WAN interface 2010 of server system 2000 is also connected. In various embodiments, network interface 2020 can include a wired interface (e.g., Ethernet) and/or a wireless interface implementing various RF data communication standards such as Wi-Fi, Bluetooth, or cellular data network standards (e.g., 3G, 4G, LTE, etc.).

User input device 2022 can include any device (or devices) via which a user can provide signals to client computing system 2014; client computing system 2014 can interpret the signals as indicative of particular user requests or information. In various embodiments, user input device 2022 can include any or all of a keyboard, touch pad, touch screen, mouse or other pointing device, scroll wheel, click wheel, dial, button, switch, keypad, microphone, and so on.

User output device 2024 can include any device via which client computing system 2014 can provide information to a user. For example, user output device 2024 can include display-to-display images generated by or delivered to client computing system 2014. The display can incorporate various image generation technologies, e.g., a liquid crystal display (LCD), light-emitting diode (LED) display including organic light-emitting diodes (OLED), projection system, cathode ray tube (CRT), or the like, together with supporting electronics (e.g., digital-to-analog or analog-to-digital converters, signal processors, or the like). Some embodiments can include a device such as a touchscreen that function as both input and output device. In some embodiments, other user output devices 2024 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When these program instructions are executed by one or more processing units, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 2004 and 2016 can provide various functionality for server system 2000 and client computing system 2014, including any of the functionality described herein as being performed by a server or client, or other functionality.

It will be appreciated that server system 2000 and client computing system 2014 are illustrative and that variations and modifications are possible. Computer systems used in connection with embodiments of the present disclosure can have other capabilities not specifically described here. Further, while server system 2000 and client computing system 2014 are described with reference to particular blocks, it is to be understood that these blocks are defined for convenience of description and are not intended to imply a particular physical arrangement of component parts. For instance, different blocks can be but need not be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including but not limited to specific examples described herein. Embodiments of the present disclosure can be realized using any combination of dedicated components and/or programmable processors and/or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware and/or software components may also be used and that particular operations described as being implemented in hardware might also be implemented in software or vice versa.

Computer programs incorporating various features of the present disclosure may be encoded and stored on various computer readable storage media; suitable media include magnetic disk or tape, optical storage media such as compact disk (CD) or digital versatile disk (DVD), flash memory, and other non-transitory media. Computer readable media encoded with the program code may be packaged with a compatible electronic device, or the program code may be provided separately from electronic devices (e.g., via Internet download or as a separately packaged computer-readable storage medium).

Thus, although the disclosure has been described with respect to specific embodiments, it will be appreciated that the disclosure is intended to cover all modifications and equivalents within the scope of the following claims.

## Claims

1. A method of providing training via visual elements for presentation to address maladaptive behavior in users, comprising:
identifying, by a computing system, a session defining a plurality of tasks for a user to be performed using at least one hand of the user holding a display to address a propensity for behavior associated with a condition of the user;
providing, by the computing system, a first indication to perform a first task of the plurality of tasks using at least one hand in a first portion of the session;
providing, by the computing system, subsequent to the first task, a second indication to perform a second task of the plurality of tasks using at least one hand in a second portion of the session; and
providing, by the computing system, subsequent to the second task, a third indication to perform a third task of the plurality of tasks using at least one hand in a third portion of the session.

2. The method of claim 1, wherein the plurality of tasks comprises one or more of (i) a go/no-go task to prompt the user to perform or withhold a first action, (ii) a habit reversal therapy task to prompt the user to perform a second action instead of performing a third action associated with the maladaptive behavior, and (iii) an urge surfing task to prompt the user to perform a fourth action for the duration of the propensity for the maladaptive behavior.

3. The method of claim 1, wherein providing the second indication further comprises providing the second indication responsive to completion of the first task, and wherein providing the third indication further comprises providing the third indication responsive to completion of the second task.

4. The method of claim 1, wherein at least one of the tasks is to perform at least one of an interaction or non-interaction with a stimulus associated with the condition, the stimulus comprising at least one of text, an image, or audio.

5. The method of claim 1, further comprising:
receiving, by the computing system, a request from the user indicating an onset of the behavior associated with the condition; and
initiating, by the computing system, responsive to receiving the request, the session to address the propensity for the behavior associated with the condition.

6. The method of claim 1, further comprising:
receiving, by the computing system, a sensory data identifying one or more physiological measurements of the user while performing the first task, and
modifying, by the computing system, the second task based on the one or more physiological measurements of the user identified in the sensory data.

7. The method of claim 6, wherein the one or more physiological measurements further comprises at least one of a breathing pattern, a pupil dilation, a heart rate, skin conductance, or oxygen saturation.

8. The method of claim 1, further comprising determining, by the computing system, a score based on performance of at least one of the tasks, the score identifying at least one of (i) a degree of compliance of the user with the at least one task or (ii) a likelihood of overcoming the propensity for the behavior.

9. The method of claim 1, further comprising generating, by the computing system, feedback to provide to the user based on performance of at least one of the tasks.

10. The method of claim 1, wherein the condition includes a skin condition corresponding to at least one of (i) atopic dermatitis, (ii) contact dermatitis, (iii) psoriasis, (iv) urticaria, (v) prurigo nodularis, (vi) lichen planus, (vii) acne vulgaris, (viii) a drug-induced rash, (xi) xerosis, or (x) uremia, wherein the user is taking a medication to address the skin condition, at least in partial concurrence with the session to address the propensity of maladaptive behavior of scratching.

11. A system for configuring visual elements to inhibit user propensity for maladaptive behavior, comprising:
a computing system having one or more processors coupled with memory, configured to perform the method of any one preceding claim.

12. A computer-readable storage medium comprising computer-readable instructions that, when executed by one or more processors, cause the one or more processors to perform the method of any one of claims 1 to 10.
